(19) Europäisches Patentamt
European Patent Office
Office européen des brevets

(11) Veröffentlichungsnummer: **0 131 793**
**B1**

(12) # EUROPÄISCHE PATENTSCHRIFT

(45) Veröffentlichungstag der Patentschrift: **25.01.89**

(21) Anmeldenummer: **84107298.6**

(22) Anmeldetag: **26.06.84**

(51) Int. Cl.⁴: **C 07 D 327/04**, C 07 D 411/06, A 01 N 43/28, A 01 N 43/84, A 01 N 43/36, A 01 N 43/40, A 01 N 43/46, A 01 N 43/60, C 07 D 413/06

(54) **5-Aminomethyl-1,3-oxathiolane.**

(30) Priorität: **08.07.83 DE 3324769**

(43) Veröffentlichungstag der Anmeldung: **23.01.85 Patentblatt 85/4**

(45) Bekanntmachung des Hinweises auf die Patenterteilung: **25.01.89 Patentblatt 89/4**

(84) Benannte Vertragsstaaten: **AT BE CH DE FR GB IT LI NL SE**

(56) Entgegenhaltungen:
**EUR. J. MED. CHEM. - CHIMICA THERAPEUTICA, Band 16, Nr. 5, September-Oktober 1981, Seiten 415-419, Paris, FR; M. PIGINI et al.: "Molecular requirements of the active sites of the cholinergic receptors XVII. 1,3-oxathiolane nucleus as a carrier of the active moieties**
**ARZNEIM.-FORSCH. (DRUG RES.), Band 25, Nr. 11, 1975, Seiten 1702-1704, Aulendorf, DE; J.G.R. ELFERINK et al.: "Relation between structure and cholinergic activity II. Synthesis and muscarinic activity of some sulfur analogs of 2-methyl-4-trimethylammoniomethyl-1,3-dioxolane-iodide"**
**Tetrahedron Letters, 23, No. 1, 47-50 (1982)**

(73) Patentinhaber: **BAYER AG, Konzernverwaltung RP Patentabteilung, D-5090 Leverkusen 1 Bayerwerk (DE)**

(72) Erfinder: **Weissmüller, Joachim, Dr., Sillerstrasse 49, D-5600 Wuppertal 11 (DE)**
Erfinder: **Krämer, Wolfgang, Dr., Am Eckbusch 39/45, D-5600 Wuppertal 1 (DE)**
Erfinder: **Berg, Dieter, Dr., Gellertweg 27, D-5600 Wuppertal 1 (DE)**
Erfinder: **Reinecke, Paul, Dr., Lessingstrasse 11, D-5090 Leverkusen 3 (DE)**
Erfinder: **Brandes, Wilhelm, Dr., Eichendorffstrasse 3, D-5653 Leichlingen (DE)**
Erfinder: **Hänssler, Gerd, Dr., Heymannstrasse 40, D-5090 Leverkusen 1 (DE)**

EP 0 131 793 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft neue 5- Aminomethyl-1,3-oxathiolane, ein Verfahren zu ihrer Herstellung und ihre Verwendung als Schädlingsbekämpfungsmittel.

Es ist bereits bekannt, daß organische Schwefelverbindungen, wie beispielsweise das Zink-ethylen-1,2-bis-(dithiocarbamat) [vergl. R.Wegler 'Chemie der Pflanzenschutz- und Schädlingsbekämpfungsmittel', Springer Verlag, Berlin, Heidelberg, New York 1970, Band 2, S.65 f] oder auch basische Alkylaminoverbindungen, wie beispielsweise das 4-[3-(4-t-Butyl-phenyl)-2-methyl]-propyl-2,6-dimethyl-morpholin (vgl. DE-OS 2 656 747) fungizide Eigenschaften besitzen.

Weiterhin ist das 5-Dimethylaminomethyl-2-methyl-1,3-oxathiolan aus einem Artikel bekannt, in dem Beziehungen zwischen Struktur und cholinergischer Aktivität untersucht werden (vgl. Arzneimittelforschung (Drug Res. ) 25, Nr. 11 (1975), 1702 - 1704).

Die Wirkung dieser Verbindungen ist jedoch insbesondere bei niedrigen Aufwandmengen und -konzentrationen in einigen Anwendungsbereichen nicht immer völlig zufriedenstellend. Es wurden neue 5-Aminomethyl-1,3-oxathiolane der allgemeinen Formel (I)

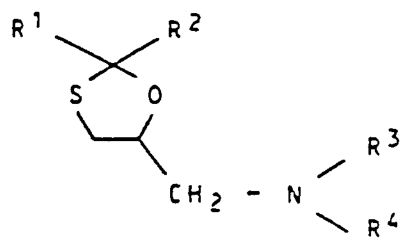

(I)

in welcher

$R_1$ für Tetrahydronaphthyl, Decahydronaphthyl oder für gegebenentalls einfach oder mehrfach, gleich oder verschieden substituiertes Naphthyl steht, wobei als Substituenten infrage kommen: Hydroxy, Halogen, jeweils geradkettiges oder verzweigtes Alkyl, Alkoxy, Alkenyloxy, Alkinyloxy oder Alkanoyloxy mit je bis zu 4 Kohlenstoffatomen in den Alkylteilen, weiterhin für jeweils gegebenenfalls einfach oder mehrfach, gleich oder verschieden durch geradkettiges oder verzweigtes Alkyl mit bis zu 4 Kohlenstoffatomen substituiertes Cycloalkyl oder Cycloalkenyl mit je 3 bis 7 Kohlenstoffatomen steht, für gegebenenfalls einfach oder mehrfach, gleich oder verschieden substituiertes Phenyl steht, oder für im Phenylkern gegebenenfalls einfach oder mehrfach, gleich oder verschieden substituiertes Phenylalkyl, Phenoxyalkyl oder Phenylthioalkyl mit jeweils bis zu 6 Kohlenstoffatomen im geradkettigen oder verzweigten Alkylteil steht, wobei als Phenylsubstituenten jeweils infrage kommen: Hydroxy, Halogen, Cyano, Nitro, jeweils geradkettiges oder verzweigtes Alkyl, Alkoxy, Alkenyloxy, Alkinyloxy und Alkylthio mit jeweils bis zu 4 Kohlenstoffatomen, Halogenalkyl, Halogenalkoxy und Halogenalkylthio mit jeweils 1 oder 2 Kohlenstoffatomen und 1 bis 5 gleichen oder verschiedenen Halogenatomen, Cycloalkyl mit 5 bis 7 Kohlenstoffatomen, geradkettiges oder verzweigtes Alkoxycarbonyl bzw. Alkanoyloxy mit jeweils bis zu 4 Kohlenstoffatomen im Alkylteil, gegebenenfalls durch Halogen, insbesondere Fluor oder Chlor oder durch geradkettiges oder verzweigtes Alkyl mit bis zu 4 Kohlenstoffatomen substituiertes Phenyl oder Phenoxy, sowie der Rest R-O-N=CH-; wobei R jeweils für geradkettiges oder verzweigtes Alkyl, Alkenyl oder Alkinyl mit bis zu 4 Kohlenstoffatomen steht; außerdem für jeweils im Cyclohexylteil gegebenenfalls einfach oder mehrfach, gleich oder verschieden durch geradkettiges oder verzweigtes Alkyl mit bis zu 4 Kohlenstoffatomen substituiertes Cyclohexylalkyl, Cyclohexyloxyalkyl oder Cyclohexylthioalkyl mit jeweils bis zu 6 Kohlenstoffatomen in den geradkettigen oder verzweigten Alkylteilen steht,

$R_2$ für Wasserstoff oder Methyl steht,

$R_3$ für geradkettiges oder verzweigtes Alkyl mit bis zu 4 Kohlenstoffatomen steht und

$R_4$ für geradkettiges oder verzweigtes Alkyl mit bis zu 4 Kohlenstoffatomen, für geradkettiges oder verzweigtes Alkenyl mit bis zu 6 Kohlenstoffatomen oder für gegebenenfalls einfach oder mehrfach gleich oder verschieden substituiertes Aralkyl mit 1 bis 2 Kohlenstoffatomen im Alkylteil und 6 bis 10 Kohlenstoffatomen im Arylteil steht, wohei als Arylsubstituenten jeweils infrage kommen: Halogen, geradkettiges oder verzweigtes Alkyl mit bis zu 4 Kohlenstoffatomen sowie Halogenalkyl mit 1 oder 2 Kohlenstoffatomen und bis zu 5 gleichen oder verschiedenen Halogenatomen oder

$R_3$ und $R_4$ zusammen mit dem Stickstoffatom, an welches sie gebunden sind, für einen gegebenenfalls einfach oder mehrfach, gleich oder verschieden substituierten 5- bis 7-gliedrigen gesättigten Heterocyclus mit 1 bis 3 Heteroatomen, vorzugsweise Stickstoff und Sauerstoff stehen, wobei als Substituenten infrage kommen: geradkettiges oder verzweigtes Alkyl mit bis zu 4 Kohlenstoffatomen, geradkettiges oder verzweigtes Alkoxycarbonyl mit bis zu 5 Kohlenstoffatomen, Phenyl, Hydroxymethyl sowie die $R'-CO-O-CH_2$-Gruppe, wobei R' für geradkettiges oder verzweigtes Alkyl, Alkoxy oder Alkylamino bzw. Dialkylamino mit jeweils 1 bis 6 Kohlenstoffatomen in den einzelnen Alkylteilen, für geradkettiges oder verzweigtes Alkoxyalkyl mit jeweils 1 bis 6 Kohlenstoffatomen in beiden Alkylteilen oder für den Furylrest steht,

sowie deren pflanzenverträglichen Säureadditionssalze und Metallsalzkomplexe gefunden.

Die Verbindungen der Formel (I) können als geometrische und/oder optische Isomere oder

Isomerengemische unterschiedlicher Zusammensetzung anfallen. Sowohl die reinen Isomeren als auch die Isomerengemische werden erfindungsgemäß beansprucht.

Weiterhin wurde gefunden, daß man die 5-Aminomethyl-1,3-oxathiolane der Formel (I) erhält, wenn man in 5-Stellung substituierte 1,3-Oxa-thiolane der allgemeinen Formel (II),

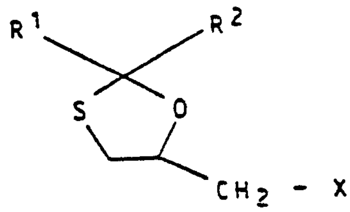

(II)

in welcher

$R^1$ und $R^2$ die oben angegebene Bedeutung haben und

x für Halogen oder für gegebenenfalls substituiertes Alkylsulfonyloxy bzw. Arylsulfonyloxy steht,

mit Aminen der allgemeinen Formel (III),

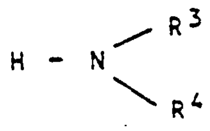

(III)

in welcher

$R^3$ und $R^4$ die oben angegebene Bedeutung haben,

gegebenenfalls in Gegenwart eines Verdünnungsmittels, gegebenenfalls in Gegenwart eines Katalysators und gegebenenfalls in Gegenwart eines Säurebindemittels umsetzt und gegebenenfalls noch anschließend eine Säure oder ein Metallsalz addiert.

Außerdem ist es möglich, die erfindungsgemäßen 5-Amino-methyl-1,3-oxathiolane nach allgemein üblichen Methoden am Stickstoff zu quarternisieren zu den entsprechenden tetrasubstituierten Ammoniumsalzen.

Die neuen 5-Aminomethyl-1,3-oxathiolane der Formel (I) weisen vor allem fungizide Eigenschaften auf. Dabei zeigen die erfindungsgemäßen Verbindungen der Formel (I) überraschenderweise eine höhere fungizide Wirksamkeit als die aus dem Stand der Technik bekannten Verbindungen Zink-ethylen-1,2-bis-(dithiocarbamat) oder 4-[3-(4-t-Butylphenyl)-2-methyl]-propyl-2,6-dimethyl-morpholin, welches Verbindungen gleicher Wirkungsrichtung sind. Die erfindungsgemäßen Verbindungen stellen somit eine Bereicherung der Technik dar.

Die erfindungsgemäßen 5-Aminomethyl-1,3-oxathiolane sind durch die Formel (I) allgemein definiert. Bevorzugt sind Verbindungen der Formel (I), bei denen

$R^1$ für Tetrahydronaphthyl, Decahydronaphthyl oder für gegebenenfalls ein- bis dreifach, gleich oder verschieden substituiertes Napnthyl steht, wobei als Substituenten infrage kommen: Fluor, Chlor, Brom, Iod, Hydroxy, Methyl, Ethyl, Methoxy, Ethoxy, Allyloxy, Propargyloxy und Acetyloxy,

weiterhin für jeweils gegebenenfalls ein- bis dreifach gleich oder verschieden durch Methyl oder Ethyl substituiertes Cycloalkyl oder Cycloalkenyl mit 5 bis 7 Kohlenstoffatomen, für gegebenenfalls ein- bis dreifach, gleich oder verschieden substituiertes Phenyl oder für einen im Phenylkern gegebenenfalls ein- bis dreifach, gleich oder verschieden substituierten Rest der Formel

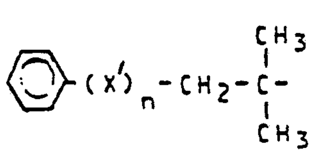 ; 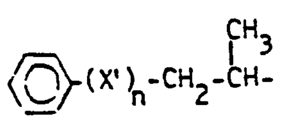 ; 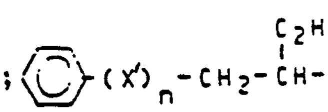 ;

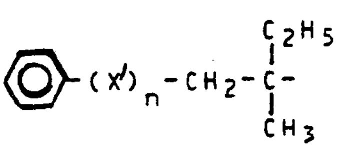 oder 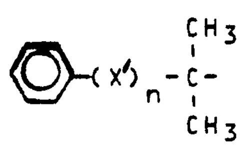 steht,

wobei als Phenylsubstituenten jeweils infrage kommen: Hydroxy, Fluor, Chlor, Brom, Iod, Cyano, Nitro, Methyl, Methoxy, Methylthio, Ethyl, Ethoxy, Ethylthio, n- und i-Propyl, Isopropyloxy, n-, i-, s- und t-Butyl, Allyloxy, Propargyloxy, Trifluormethyl, Trifluormethoxy, Trifluormethylthio, Cyclohexyl, Methoxycarbonyl, Ethoxycarbonyl, Acetyloxy, gegebenenfalls durch Fluor, Chlor oder Methyl substituiertes Phenyl oder Phenoxy, sowie der Rest R-O-N=CH-, wobei R jeweils für Methyl, Ethyl, n- und i-Propyl, n-, i-, s- und t-Butyl, Allyl und

Propargyl steht; außerdem für einen im Cyclohexylteil gegebenenfalls ein- bis dreifach, gleich oder verschieden durch Methyl, Ethyl oder Isopropyl substituierten Rest der Formel

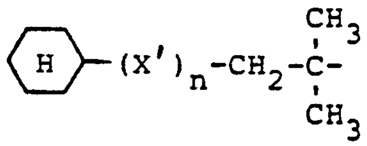 ; 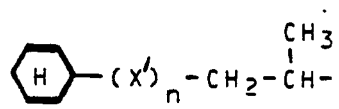 ;

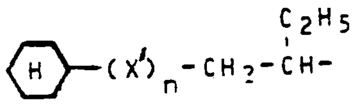 ; 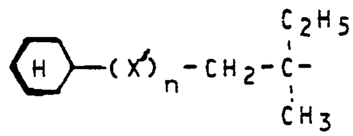 oder

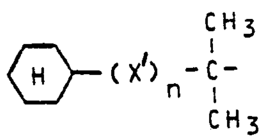 steht, wobei

steht, wobei

in allen oben formelmäßig dargestellten Resten

X' jeweils für Sauerstoff oder Schwefel und n für 0 oder 1 steht,

$R^2$ für Methyl steht,

$R^3$ für Methyl, Ethyl, n- und i-Propyl steht und

$R^4$ für Methyl, Ethyl, n- und i-Propyl, n-, i-, s- und t-Butyl, Allyl, 2-Butenyl, 3-Methyl-2-butenyl sowie für gegebenenfalls ein- bis dreifach, gleich oder verschieden durch Fluor, Chlor, Methyl oder Trifluormethyl substituiertes Benzyl steht oder

$R^3$ und $R^4$ zusammen mit dem Stickstoffatom, an welches sie gebunden sind, für jeweils gegebenenfalls ein- bis dreifach, gleich oder verschieden substituiertes 1-Pyrrolidinyl, 1-Piperidinyl, 1-Piperazinyl, 4-Morpholinyl oder 1-Perhydroazepinyl stehen, wobei als Substituenten genannt seien: Methyl, Ethyl, n- und i-Propyl, Phenyl, Hydroxymethyl, Acetyloxymethyl, Methoxycarbonyloxymethyl, Dimethylaminocarbonyloxymethyl, Diethylaminocarbonyloxymethyl, Methoxyacetyloxymethyl oder Furoyloxymethyl.

Im einzelnen seien außer den bei den Herstellungsbeispielen genannten Verbindungen die folgenden Verbindungen der allgemeinen Formel (I) genannt:

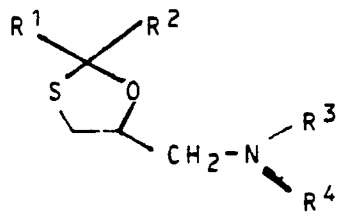 (I)

| $R^1$ | $R^2$ | 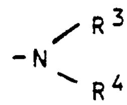 |
|---|---|---|
| 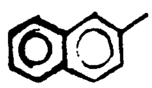 | $CH_3$ | 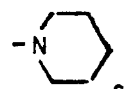 |
| 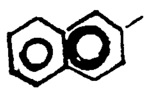 | $CH_3$ | 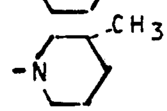 |
| 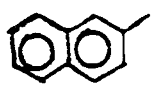 | $CH_3$ | 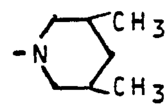 |
| 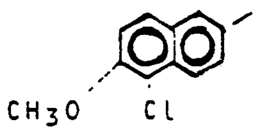 | $CH_3$ | 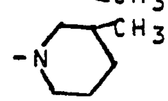 |
| 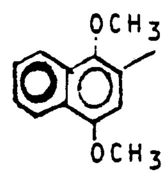 | $CH_3$ | 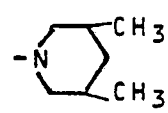 |

| $R^1$ | $R^2$ | 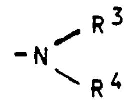 |
|---|---|---|
| 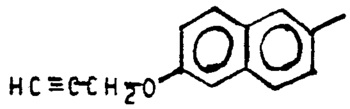 | $CH_3$ | 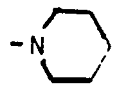 |
| 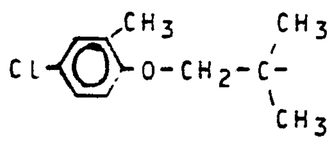 | $CH_3$ | 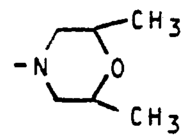 |
| 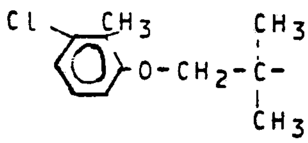 | $CH_3$ | 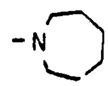 |
| 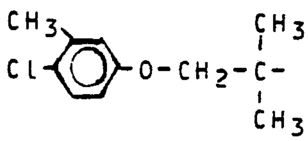 | $CH_3$ | 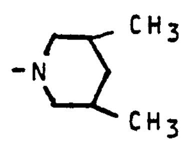 |
| 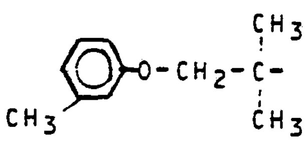 | $CH_3$ | 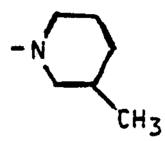 |
| 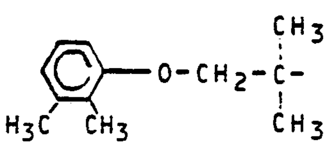 | $CH_3$ | 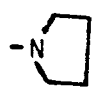 |
| 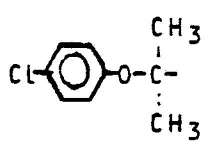 | $CH_3$ | 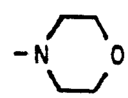 |

| $R^1$ | $R^2$ | 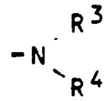 |
|---|---|---|
| 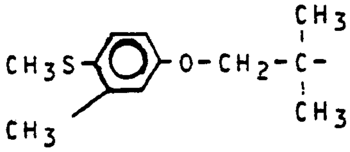 | $CH_3$ | 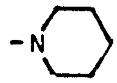 |
| 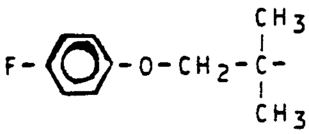 | $CH_3$ | 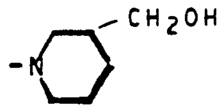 |
| 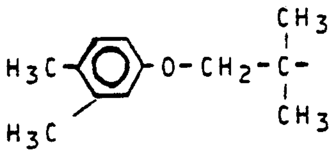 | $CH_3$ | 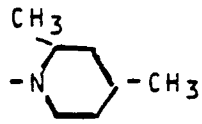 |
| 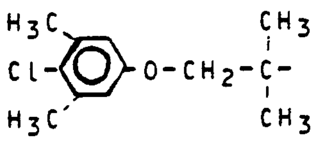 | $CH_3$ | 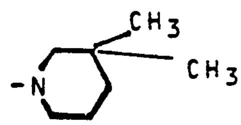 |
| 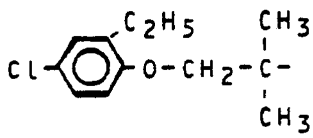 | $CH_3$ | 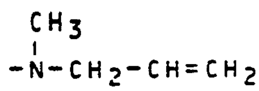 |
| 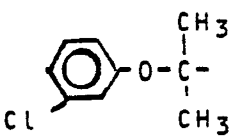 | $CH_3$ | 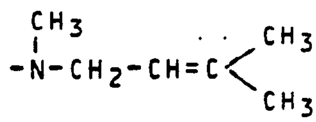 |
| 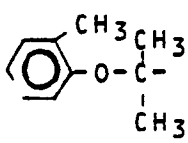 | $CH_3$ | 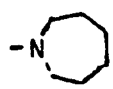 |
| 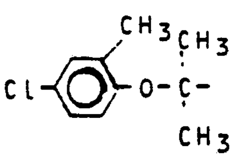 | $CH_3$ | 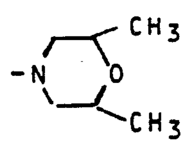 |
| 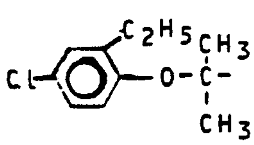 | $CH_3$ | 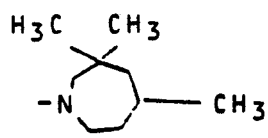 |
| 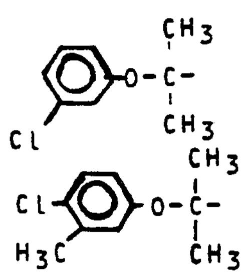 | $CH_3$ | 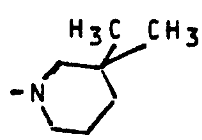 |
| | $CH_3$ | 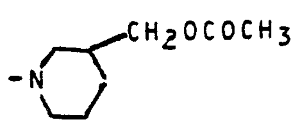 |

| $R^1$ | $R^2$ | 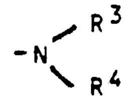 |
|---|---|---|
| 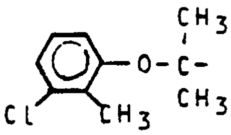 | $CH_3$ | 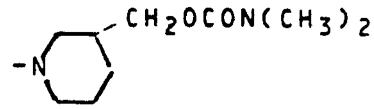 |
| 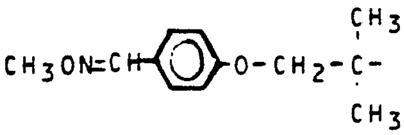 | $CH_3$ | 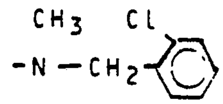 |
| 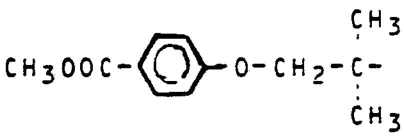 | $CH_3$ | 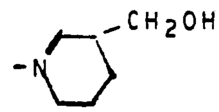 |
| 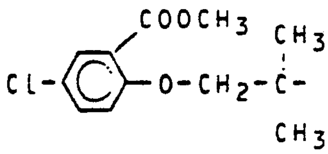 | $CH_3$ | 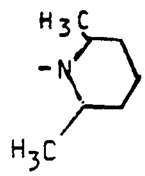 |
| 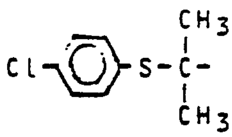 | $CH_3$ | 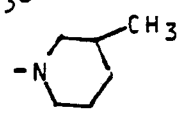 |
| 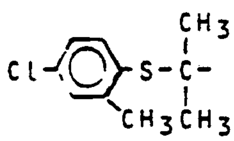 | $CH_3$ | 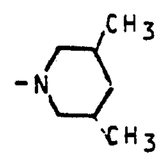 |
| 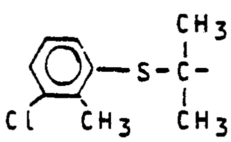 | $CH_3$ | 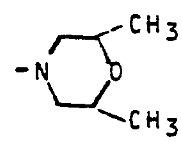 |
| 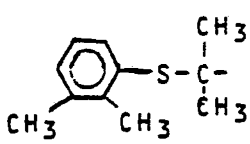 | $CH_3$ | 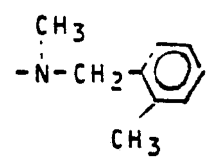 |
| 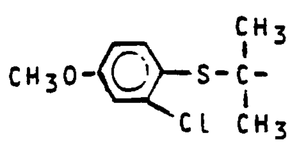 | $CH_3$ | 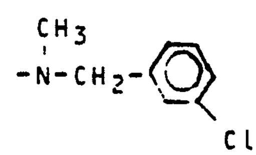 |

| $R^1$ | $R^2$ | -N $R^3$ $R^4$ |
|---|---|---|
| 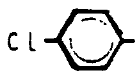 | $CH_3$ | 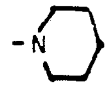 |
| 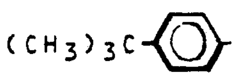 | $CH_3$ | 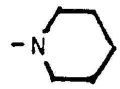 |
| 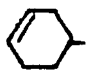 | H | 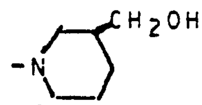 |
| 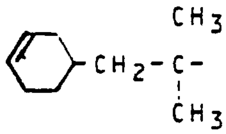 | $CH_3$ | 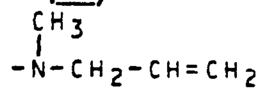 |
| 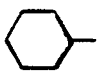 | $CH_3$ | 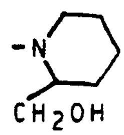 |
| 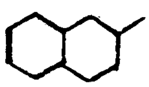 | $CH_3$ | 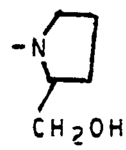 |
| 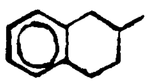 | $CH_3$ | 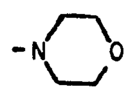 |
| 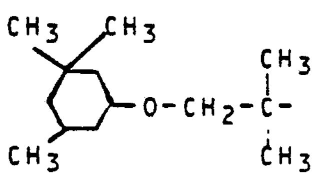 | $CH_3$ | 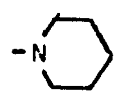 |
| 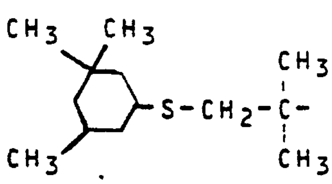 | $CH_3$ | 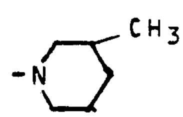 |
| 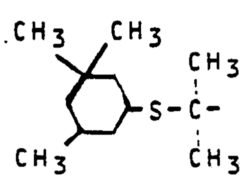 | $CH_3$ | 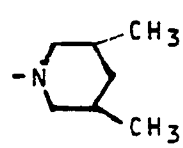 |

| $R^1$ | $R^2$ | 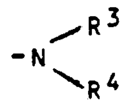 |
|---|---|---|
| 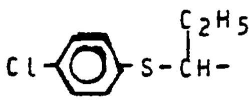 | $CH_3$ | 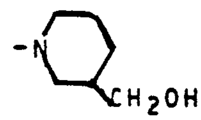 |
| 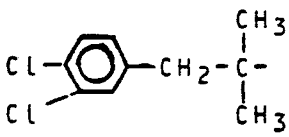 | $CH_3$ | 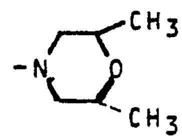 |
| 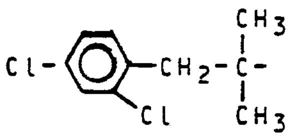 | $CH_3$ | 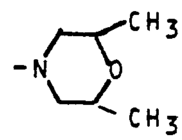 |
| 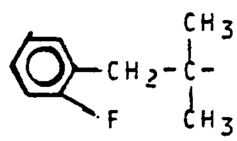 | $CH_3$ | 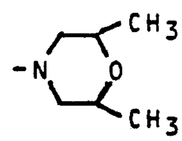 |
| 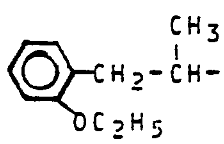 | $CH_3$ | 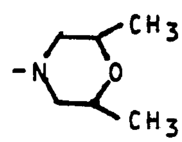 |
| 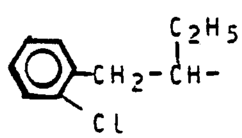 | $CH_3$ | 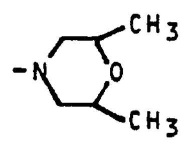 |
| 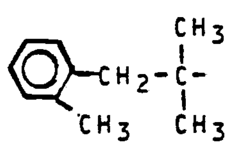 | $CH_3$ | 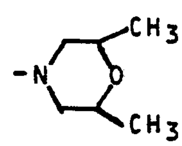 |
| 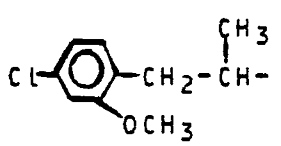 | $CH_3$ | 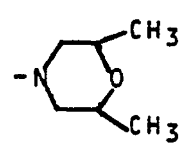 |

Verwendet man beispielsweise 5-Chlormethyl-2-[1-(4-chlor-phenoxy)-2-methyl]-prop-2-yl-2-methyl-1,3-oxathiolan und Piperidin als Ausgangsstoffe, so kann der Reaktionsablauf des erfindungsgemäßen Verfahrens durch das folgende Formelschema wiedergegeben werden:

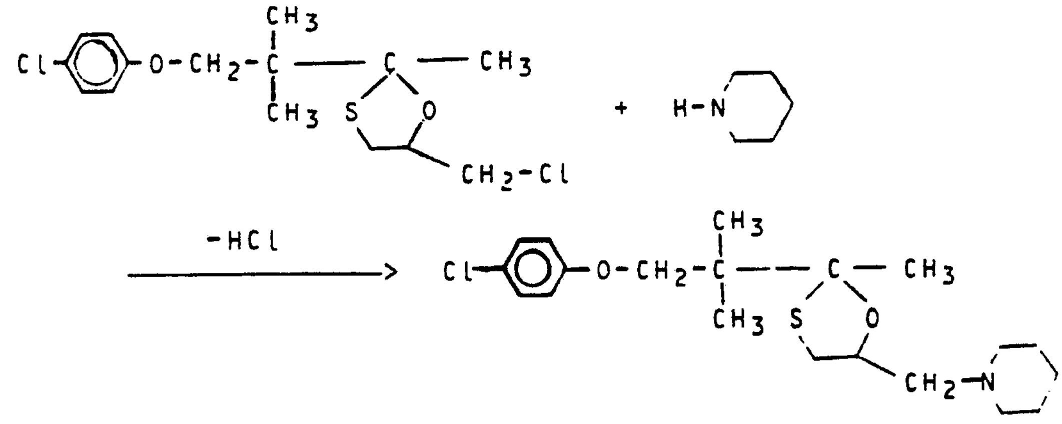

Die zur Durchführung des erfindungsgemäßen Verfahrens als Ausgangsstoffe benötigten in 5-Stellung substituierten 1,3-Oxathiolane sind durch die Formel (II) allgemein definiert. In dieser Formel besitzen $R^1$ und $R^2$ vorzugsweise die gleiche Bedeutung, die bei der Beschreibung der erfindungsgemäßen Verbindungen der Formel (I) als bevorzugt angegeben wird X steht vorzugsweise für Chlor, Brom, Methansulfonyloxy, Trifluormethansulfonyloxy oder p-Toluolsulfonyloxy.

Die in 5-Stellung substituierten 1,3-Oxathiolane der Formel (II) sind teilweise bekannt (vergleiche Tetrahedron Letters 23, 47-50 [1982]).

Noch nicht bekannt sind Verbindungen der Formel (IIa)

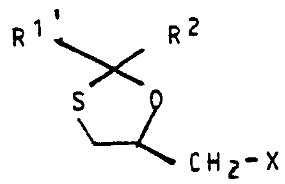

(IIa)

in welcher

$R^{1'}$ für Tetrahydronaphthyl, Decahydronaphthyl oder für gegebenenfalls substituiertes Naphthyl, für gegebenenfalls substituiertes Cycloalkyl, für substituiertes Phenyl oder für durch - jeweils gegebenenfalls substituiertes - Phenyl, Phenoxy, Phenylthio, Cyclohexyl, Cyclohexyloxy oder Cyclohexylthio substituiertes Alkyl steht,

$R^2$ für Wasserstoff oder Methyl steht und

X für Halogen oder für gegebenenfalls substituiertes Alkylsulfonyloxy bzw. Arylsulfonyloxy steht.

Man erhält die Verbindungen der Formel (IIa) in prinzipiell bekannter Weise, wenn man Aldehyde oder Ketone der Formel (IV),

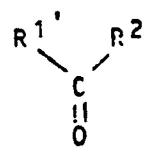

(IV)

in welcher

$R^{1'}$ und $R^2$ die oben angegebene Bedeutung haben, mit Thiopropanol-Derivaten der Formel (V),

$$HS-CH_2-CH(OH)-CH_2-Y \quad (V)$$

in welcher

Y für Halogen oder Hydroxy steht,

gegebenenfalls in Gegenwart eines Verdünnungsmittels, wie beispielsweise Diethylether und gegebenenfalls in Gegenwart eines Katalysators, wie beispielsweise Bortrifluorid bei Temperaturen zwischen

0°C und 120°C umsetzt, und in den Fällen, wo Y für die Hydroxygruppe steht, die so erhaltenen 5-Hydroxymethyl-1,3-oxathiolane der Formel (IIb),

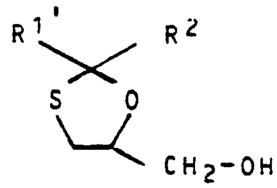

(IIb)

in welcher

$R^{1'}$ und $R^2$ die oben angegebene Bedeutung haben,

mit gegebenenfalls substituierten Alkyl- bzw. Arylsulfonylhalogeniden der Formel (VI),

$$Z - SO_2 - Hal \qquad (VI)$$

in welcher

Hal für Halogen, vorzugsweise für Chlor, steht und

Z für gegebenenfalls substituiertes Alkyl bzw. Aryl, vorzugsweise für Methyl, Trifluormethyl oder 4-Methylphenyl, steht

gegebenenfalls in Gegenwart eines Verdünnungsmittels, wie beispielsweise Diethylether, und gegebenenfalls in Gegenwart eines Säurebindemittels, wie beispielsweise Pyridin oder Triethylamin bei Tenperaturen zwischen -20°C und +100°C umsetzt zu den Verbindungen der Formel (IIc),

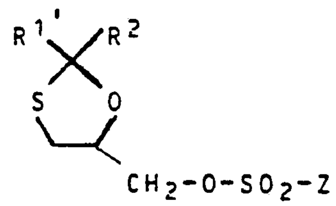

(IIc)

in welcher

$R^{1'}$, $R^2$ und Z die oben angegebene Bedeutung haben.

Die Ketone der Formel (IV) sind bekannt (vgl. z. B. DE-OS 3 210 725 oder DE-0S 3 048 266) oder können nach bekannten Methoden in einfacher Weise erhalten werden.

Die Thiopropanolderivate der Formel (V) und die Sulfonylhalogenide der Formel (VI) sind allgemein bekannte Verbindungen der organischen Chemie.

Die außerdem für das erfindungsgemäße Verfahren als Ausgangsstoffe benötigten Amine sind durch die Formel (III) allgemein definiert. In dieser Formel stehen $R^3$ und $R^4$ vorzugsweise für diejenigen Reste, die bereits im Zusammenhang mit der Beschreibung der erfindungsgemäßen Stoffe der Formel (I) vorzugsweise für diese Substituenten genannt wurden.

Die Amine der Formel (III) sind allgemein bekannte Verbindungen der organischen Chemie.

Das erfindungsgemäße Verfahren kann gegebenenfalls in Gegenwart eines Verdünnungsmittels durchgeführt werden. Als Verdünnungsmittel kommen inerte organische Lösungsmittel infrage. Hierzu gehören vorzugsweise aromatische Kohlenwasserstoffe, wie Benzol, Toluol oder Xylol; halogenierte Kohlenwasserstoffe, wie Tetrachlorkohlenstoff oder Chlorbenzol; Formamide, wie Dimethylformamid; Nitrile, wie Acetonitril oder Propionitril; Alkohole, wie Propanol oder Butanol; Amine, wie Triethylamin oder Piperidin; sowie die hochpolaren Lösungsmittel Dimethylsulfoxid oder Hexamethylphosphorsäuretriamid.

Das erfindungsgemäße Verfahren wird gegebenenfalls in Gegenwart einer Base als Säurebindemittel durchgeführt. Hierbei können alle üblichen organischen und insbesondere anorganische Basen eingesetzt werden. Hierzu gehören vorzugsweise Alkalimetallhydroxide oder Carbonate, wie beispielsweise Natriumhydroxid, Natriumcarbonat oder Kaliumcarbonat; ferner Triethylamin und Pyridin. Gegebenenfalls können auch die als Ausgangsstoffe eingesetzten Amine der Formel (III) als Verdünnungsmittel und als Säurebindemittel verwendet werden.

Das erfindungsgemäße Verfahren wird gegebenenfalls in Gegenwart eines Katalysators durchgeführt. Vorzugsweise verwendet man Alkalimetalliodide, wie zum Beispiel Kaliumiodid.

Die Reaktionstemperaturen können bei dem erfindungsgemäßen Verfahren in einem größeren Bereich variiert werden. Im allgemeinen arbeitet man zwischen 50°C und 250°C, vorzugsweise zwischen 80°C und 200°C.

Das erfindungsgemäße Verfahren kann bei Normaldruck, aber auch bei erhöhtem Druck durchgeführt

werden. Unter Druck arbeitet man im allgemeinen zwischen etwa 1,5 atü und 5 atü, vorzugsweise zwischen 1,5 atü und 3 atü.

Bei der Durchführung des erfindungsgemäßen Verfahrens setzt man auf 1 Mol substituiertes Oxathiolan der Formel (II) vorzugsweise 1 bis 30 Mol Amin der Formel (III) ein, je nachdem ob das Amin auch als Verdünnungsmittel und/oder Säurebindemittel verwendet wird. Die Isolierung der Endprodukte erfolgt nach üblichen Methoden.

Zur Herstellung von pflanzenverträglichen Säureadditionssalzen der Verbindungen der Formel (I) kommen vorzugsweise folgende Säuren infrage: Die Halogenwasserstoffsäuren, wie z. B. die Chlorwasserstoffsäure und die Bromwasserstoffsäure, insbesondere die Chlorwasserstoffsäure, fefner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure, Milchsäure, sowie Sulronsäuren, wie z. B. p-Toluolsulfonsäure und 1,5-Naphthalindisulfonsäure. Die Säureadditionssalze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden. Zur Herstellung von Metallsalz-Komplexen der Verbindungen der Formel (I) kommen vorzugsweise Salze von Metallen der II. bis IV. Haupl- und der I. oder II. sowie IV. bis VIII. Nebengruppe infrage, wobei Kupfer, Zink, Mangan, Magnesium, Zinn, Eisen und Nickel beispielhaft genannt seien. Als Anionen der Salze kommen solche in Betracht, die sich vorzugsweise von folgenden Säuren ableiten: Halogenwasserstoffsäuren, wie z. B. die Chlorwasserstoffsäure und die Bromwasserstoffsäure, ferner Phosphorsäure, Salpetersäure und Schwefelsäure. Die Melallsalz-Komplexe von Verbindungen der Formel (I) könnnen in einfacher Weise nach üblichen Verfahren erhalten werden, so z. B. durch Lösen des Metallsalzes in Alkohol, z. B. Ethanol und Hinzufügen zur Verbindung der Formel (I). Man kann Metallsalz-Komplexe in bekannter Weise, z. B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Schädlingsbekämpfungsmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomyceles, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pifanzenschutzmittel können die erfindungsgemäßen Wirkstoffe mit besonders gutem Erfolg zur Bekämpfung von Getreidekrankheiten, wie beispielsweise gegen den Erreger des Gerstenmehltaus (Erysiphe graminis) oder gegen den Erreger der Gerstenstreifenkrankheit (Drechslera graminea), von Reiskrankheiten, wie beispielsweise gegen den Erreger der Reisfleckenkrankheit (Pyricularia oryzae) oder von Obst- und Gemüsekrankheiten, wie beispielsweise gegen den Erreger des Apfelschorfes (Venturia inaequalis) oder gegen den Erreger des Bohnengrauschimmels (Botrytis cinerea) eingesetzt werden. Dabei besitzen die erfindungsgemäßen Wirkstoffe sowohl protektive als auch systemische Wirksamkeit.

Darüberhinaus zeigen sie auch gute Wirkung gegen die Getreidekrankheitserreger Septoria nodorum, Cochliobolus sativus und Pyrenophora teres, gegen die Reiskrankheitserreger Pellicularia sasakii, gegen Oomyceten sowie gegen echte Mehltaupilze. Daneben zeigen die erfindungsgemäßen Wirkstoffe auch eine gute fungizide in vitro Wirkung.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur-und synthetische Stoffe, Feinstverkspselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und /oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen infrage: Aromaten, wie Xylol, Toluol, oder Alkyl-naphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B.

gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier- und/oder schaumerzeugende Mittel kommen in Frage: z. B. nichtionogene und anionische Emulgatoren, wie Polyoxyethylen-Fettsäureester, Polyoxyethylen-Fettalkohol-ether, z. B. Alkylarylpolyglykol-ether, Alkylsulfonate, Alkylsulfate, Arylsulfonate sowie Eiweißhydrolysate; als Dispergiermittel kommen infrage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Herstellungsbeispiele:

**Beispiel 1**

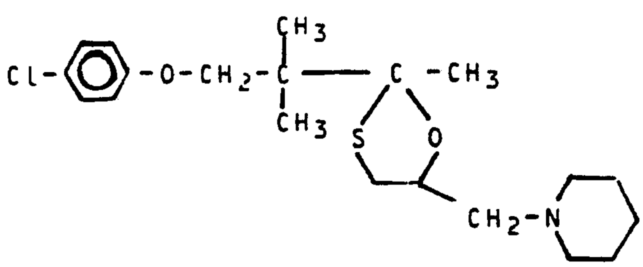

10g (0,03 Mol) 5-Chlormethyl-2-[1-(4-chlorphenoxy)-2-methyl]-prop-2-yl-2-methyl-1,3-oxathiolan und 10g (0,12 Mol) Piperidin werden 12 Stunden lang auf 120°C erhitzt. Nach dem Erkalten wird das Reaktionsgemisch mit Essigester verdünnt, zweimal mit Wasser gewaschen, über Natriumsulfat getrocknet und im Vakuum eingeengt. Der Rückstand wird einer Kugelrohrdestillation unterzogen (Kp:~200°C/0,13 mbar) oder über eine Kieselgel -Säule mit einem Laufmittelgemisch aus Petrolether/Essigester 2 : 1 chromatographiert. Man erhält 8g (69,5 % der Theorie) an 2-[1-(4-Chlorphenoxy)-2-methyl]-prop-2-yl-2-methyl-5-piperidin-1-ylmethyl-1,3-oxathiolan vom Brechungsindex $n^{20}_D = 1,5440$.

In entsprechender Weise und gemäß den allgemeinen Angaben zur Herstellung erhält man die nachfolgend aufgeführten Verbindungen der allgemeinen Formel (I):

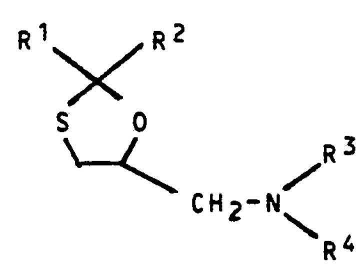

(I)

| Beispiel-Nummer | $R^1$ | $R^2$ | 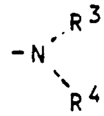 | Brechungsindex[$n_D^{20}$] |
|---|---|---|---|---|
| 2 | 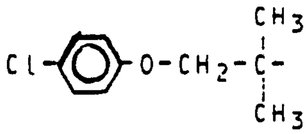 | $CH_3$ | 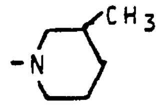 | 1,5382 |
| 3 | 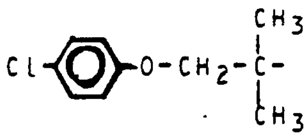 | $CH_3$ | 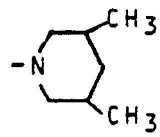 | 1,5331 |
| 4 | 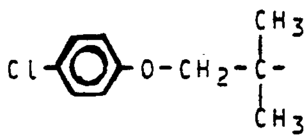 | $CH_3$ | 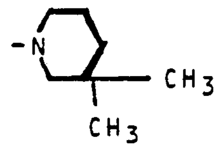 | 1,5357 |
| 5 | 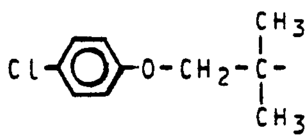 | $CH_3$ | 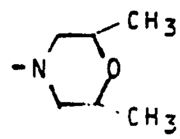 | 1,5341 |
| 6 | 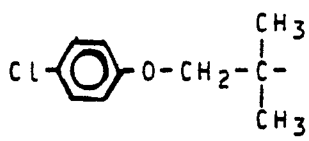 | $CH_3$ | 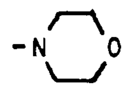 | 1,5039 |
| 7 | 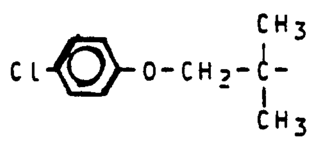 | $CH_3$ | 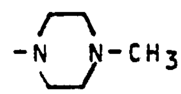 | 1,5065 |
| 8 | 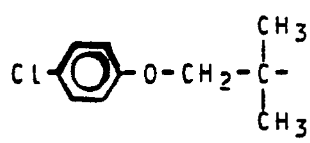 | $CH_3$ | 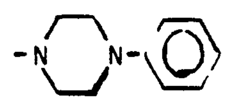 | 1,5147 |
| 9 | 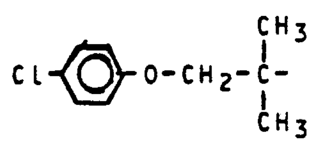 | $CH_3$ | 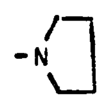 | 1,5007 |

| Bsp. Nr. | $R^1$ | $R^2$ | 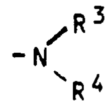 | Brechungs-index [$n_D^{20}$] |
|---|---|---|---|---|
| 10 | 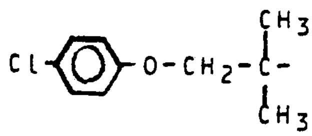 | $CH_3$ | 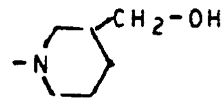 | 1,5077 |
| 11 | 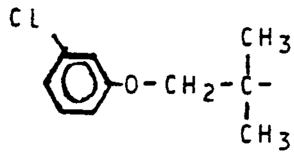 | $CH_3$ | 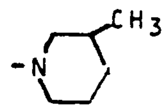 | 1,5082 |
| 12 | 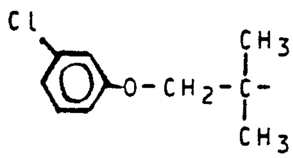 | $CH_3$ | 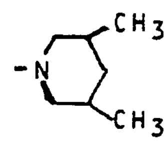 | 1,5049 |
| 13 | 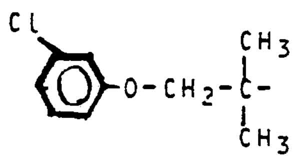 | $CH_3$ | 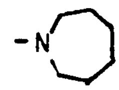 | 1,5058 |
| 14 | 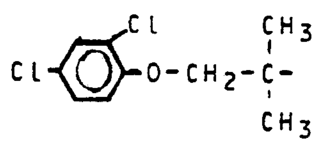 | $CH_3$ | 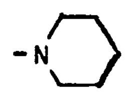 | 1,5091 |
| 15 | 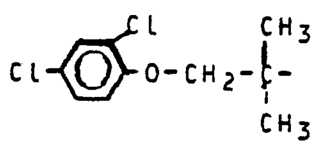 | $CH_3$ | 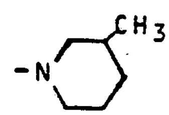 | 1,5077 |
| 16 | 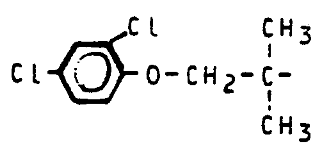 | $CH_3$ | 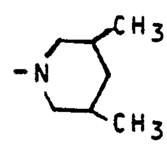 | 1,5069 |
| 17 | 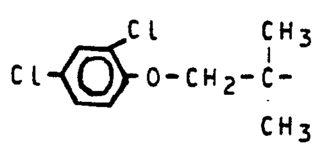 | $CH_3$ | 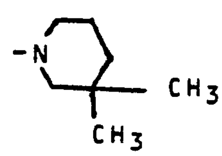 | 1,5087 |

| Bsp. Nr. | $R^1$ | $R^2$ | 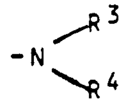 | Brechungsindex $[n_D^{20}]$ |
|---|---|---|---|---|
| 18 | 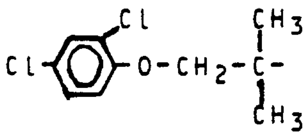 | $CH_3$ | 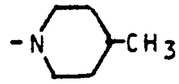 | 1,5083 |
| 19 | 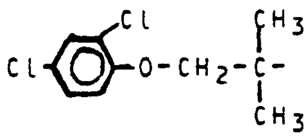 | $CH_3$ | 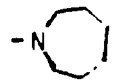 | 1,5110 |
| 20 | 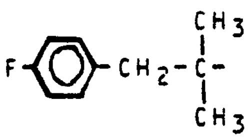 | $CH_3$ | 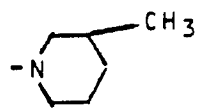 | 1,5245 |
| 21 | 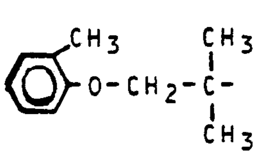 | $CH_3$ | 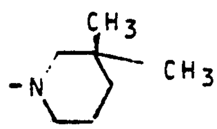 | 1,5070 |
| 22 | 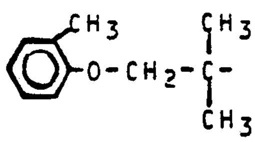 | $CH_3$ | 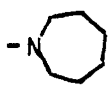 | 1,5062 |
| 23 | 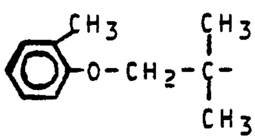 | $CH_3$ | 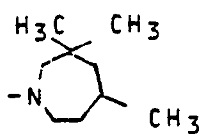 | 1,5078 |
| 24 | 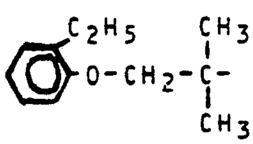 | $CH_3$ | 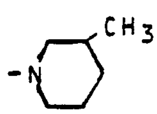 | 1,5122 |
| 25 | 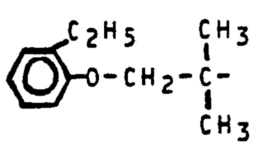 | $CH_3$ | 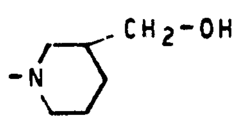 | 1,5047 |

| Bsp. Nr. | $R^1$ | $R^2$ | $-N{<}^{R^3}_{R^4}$ | Brechungsindex [$n_D^{20}$]; Schmelzpunkt [°C] |
|---|---|---|---|---|
| 26 | 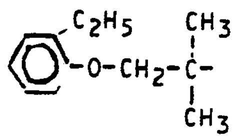 | $CH_3$ | 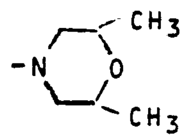 | 1,5086 (cis-Form) |
| 27 | 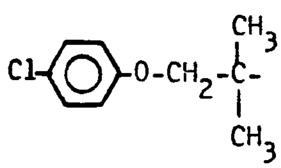 | $CH_3$ | 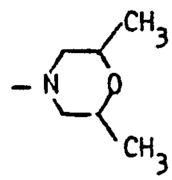 | 1,5039 (cis-Form) |
| 28 | 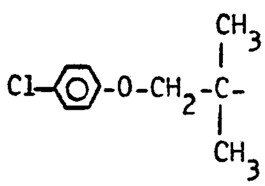 | $CH_3$ | 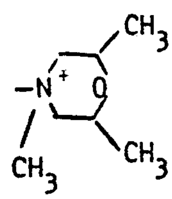 | 179-180 (Iodid) |
| 29 | 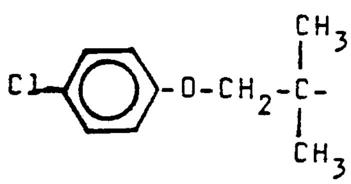 | $CH_3$ | 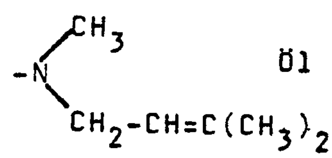 | Öl |

Herstellung der Ausgangsverbindungen:

**Beispiel II-1**

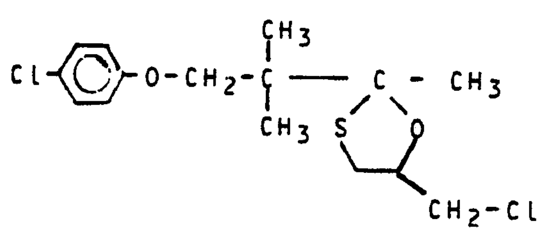

Zu einer siedenden Lösung von 80,7g (0,35 Mol) 4-(4-Chlorphenoxy)-3,3-dimethyl-butan-2-on und 45g (0,35 Mol) 1-Clor-3-mercapto-2-propanol in 240 ml absolutem Ether tropft man 50g (0,35 Mol) Bortrifluorid-Etherat. Nach beendeter Zugabe kocht man weitere 90 Minuten am Rückfluß. Die abgekühlte Reaktionsmischung wird 2 mal mit je 100 ml 0,1 molarer Natriumhydrogencarbonat- und einmal mit gesättiger Natriumchloridlösung gewaschen, über Natriumsulfat getrocknet und im Vakuum vom Lösungsmittel befreit. Der Rückstand wird im Vakuum destilliert. Man erhält 50g (43 % der Theorie) an 5-Chlormethyl-2[1-(4-chlorphenoxy)-2-methyl]-prop-2-yl-2-methyl-1,3-oxathiolan vom Siedepunkt 160° C bei 0,3 mbar.

In entsprechender Weise erhält man die folgenden Verbindungen der allgemeinen Formel (II):

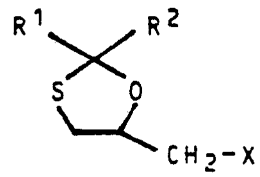

(II)

Beispiel II-2:

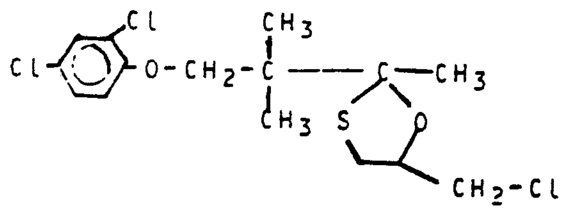

Siedepunkt: 200° C bei 0,13 mbar

Beispiel II-3:

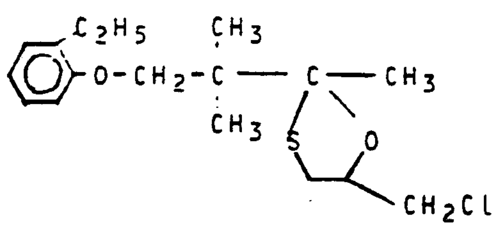

Siedepunkt: 170° C bei 0,13 mbar

Beispiel II-4:

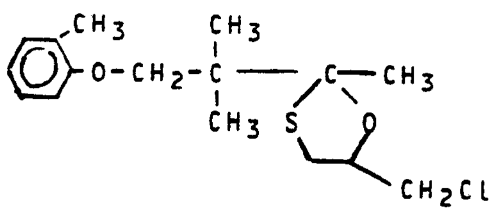

Siedepunkt: 165-170° C bei 0,13 mbar

Beispiel II-5:

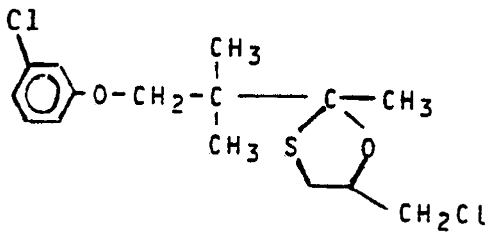

Siedepunkt: 165-190° C bei 0,26 mbar
(Kugelrohrdestillation bei 210° C)

Beispiel II-6:

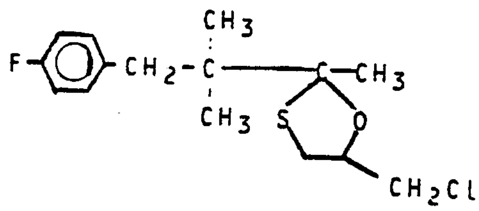

Siedepunkt: 144° C bei 0,13 mbar

Anwendungsbeispiele:

In den nachfolgenden Anwendungsbeispielen werden die nachstehend angegebenen Verbindungen als Vergleichssubstanzen eingesetzt:

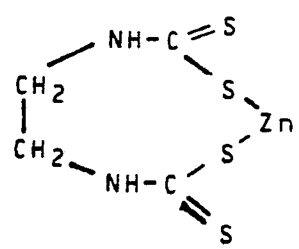

(A)

Zink-ethylen-1,2-bis-(dithiocarbamat)

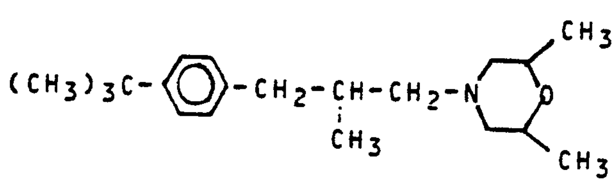

(B)

4-[3-(4-t-Butylphenyl)-2-methyl]-prop-1-yl-2,6-dimethyl-morpholin

**Beispiel A**

Erysiphe-Test (Gerste) / protektiv /

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.
Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.
Die Pflanzen werden in einem Gewächshaus bei einer Temperetur von ca. 20°C und einer relativen Luftfeuchtigkeit von ca. 80 % aufgestellt, um die Entwicklung von Mehltaupusteln zu begünstigen.
7 Tage nach der Inokulation erfolgt die Auswertung.
Eine deutliche Überlegenheit in der Wirksamkeit gegenüber dem Stand der Technik zeigen in diesem Test z. B. die Verbindungen gemäß folgender Herstellungsbeispiele: 1,2,3,4,5,6,7,11,12,14,15,16,17,18,19,21,22,23.

**Beispiel B**

Drechslera graminea-Test (Gerste) / Saatgubehandlung (syn. Helminthosporium gramineum)
Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.
Zur Beizung schüttelt man das infizierte Saatgut 3 Minuten lang mit dem Beizmittel in einer verschlossenen Glasflasche.
Das Saatgut setzt man in gesiebter, feuchter Standarderde eingebettet, in verschlossenen Petrischalen im Kühlschrank 10 Tage lang einer Temperatur von 4°C aus. Dabei wird die Keimung der Gerste und gegebenenfalls auch der Pilzsporen eingeleitet. Anschließend sät man die vorgekeimte Gerste mit 2 x 50 Korn 3 cm tief in eine Standarderde und kultiviert sie im Gewächshaus bei einer Temperatur von ca. 18°C in Saatkästen, die täglich 15 Stunden dem Licht ausgesetzt werden.
Ca. 3 Wochen nach der Aussaat erfolgt die Auswertung der Pflanzen auf Symptome der Streifenkrankheit.
Eine deutliche Überlegenheit in der Wirksamkeit gegenüber dem Stand der Technik zeigen in diesem Test z. B. die Verbindungen gemäß folgender Herstellungsbeispiele: 1 und 2.

**Beispiel C**

Venturia-Test (Apfel) / protektiv /

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.
Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in einer Inkubationskabine.
Die Pflanzen werden dann im Gewächshaus bei 20°C und einer relativen Luftfeuchtigkeit von ca. 70 % aufgestellt.
12 Tage nach der Inokulation erfolgt die Auswertung.
Eine deutliche Überlegenheit in der Wirksamkeit gegenüber dem Stand der Technik zeigen in diesem Test z. B. die Verbindungen gemäß folgender Herstellungsbeispiele: 1, 3, 5, 6 und 22.

**Beispiel D**

Botrytis-Test (Bohne) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirksfoff mit den angegebenen Mengen Lösungsmittel und Emulgator und verdünnt das Konzentrat mit Wasser auf die gewünschte Konzentration.

Zur Prüfung auf protektive Wirksankeit bespritzt man junge Pflanzen mit der Wirkstoffzubereitung bis fur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

Eine deutliche Überlegenheit in Wirksamkeit gegenüber dem Stand der Technik zeigen in diesem Test z. B. die Verbindungen gemäß folgender Herstellungsbeispiele: 5,6,14,15,18,19.

**Beispiel E**

Pyricularia-Test (Reis) / systemisch

Lösungsmittel: 12,5 Gewichtsteile Aceton
Emulgaror: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z. B. die Verbindungen gemäß folgender Herstellungsbeispiele: 2,3,4 und 23.

**Patentansprüche**

1. 5-Aminomethyl-1,3-oxathiolane der allgemeinen Formel (I)

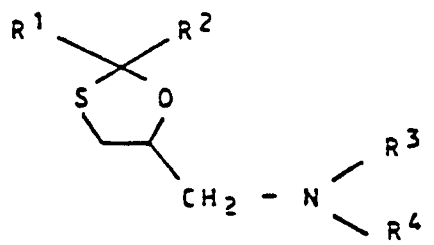

(I)

in welcher

$R^1$ für Tetrahydronaphthyl, Decahydronaphthyl oder für gegebenentalls einfach oder mehrfach, gleich oder verschieden substituiertes Naphthyl steht, wobei als Substituenten infrage kommen: Hydroxy, Halogen, jeweils geradkettiges oder verzweigtes Alkyl, Alkoxy, Alkenyloxy, Alkinyloxy oder Alkanoyloxy mit je bis zu 4 Kohlenstoffatomen in den Alkylteilen, weiterhin für jeweils gegebenenfalls einfach oder mehrfach, gleich oder verschieden durch geradkettiges oder verzweigtes Alkyl mit bis zu 4 Kohlenstoffatomen substituiertes Cycloalkyl oder Cycloalkenyl mit je 3 bis 7 Kohlenstoffatomen steht, für gegebenenfalls einfach oder mehrfach, gleich oder verschieden substituiertes Phenyl steht, oder für im Phenylkern gegebenenfalls einfach oder mehrfach, gleich oder verschieden substituiertes Phenylalkyl, Phenoxyalkyl oder Phenylthioalkyl mit jeweils bis zu 6 Kohlenstoffatomen im geradkettigen oder verzweigten Alkylteil steht, wobei als Phenylsubstituenten jeweils infrage kommen: Hydroxy, Halogen, Cyano, Nitro, jeweils geradkettiges oder verzweigtes Alkyl, Alkoxy, Alkenyloxy, Alkinyloxy und Alkylthio mit jeweils bis zu 4 Kohlenstoffatomen, Halogenalkyl,

Halogenalkoxy und Halogenalkylthio mit jeweils 1 oder 2 Kohlenstoffatomen und 1 bis 5 gleichen oder verschiedenen Halogenatomen, Cycloalkyl mit 5 bis 7 Kohlenstoffatomen, geradkettiges oder verzweigtes Alkoxycarbonyl bzw. Alkanoyloxy mit jeweils bis zu 4 Kohlenstoffatomen im Alkylteil, gegebenenfalls durch Halogen, insbesondere Fluor oder Chlor oder durch geradkettiges oder verzweigtes Alkyl mit bis zu 4 Kohlenstoffatomen substituiertes Phenyl oder Phenoxy, sowie der Rest R-O-N=CH-, wobei R jeweils für geradkettiges oder verzweigtes Alkyl, Alkenyl oder Alkinyl mit bis zu 4 Kohlenstoffatomen steht; außerdem für jeweils im Cyclohexylteil gegebenenfalls einfach oder mehrfach, gleich oder verschieden durch geradkettiges oder verzweigtes Alkyl mit bis zu 4 Kohlenstoffatomen substituiertes Cyclohexylalkyl, Cyclohexyloxyalkyl oder Cyclohexylthioalkyl mit jeweils bis zu 6 Kohlensstoffatomen in den geradkettigen oder verzweigten Alkylteilen steht,

$R^2$ für Wasserstoff oder Methyl steht,

$R^3$ für geradkettiges oder verzweigtes Alkyl mit zu 4 Konlenstoffatomen steht und

$R^4$ für geradkettiges oder verzweigtes Alkyl mit bis zu 4 Kohlenstoffatomen, für geradkettiges oder verzweigtes Alkenyl mit bis zu 6 Kohlenstoffatomen oder für gegebenenfalls einfach oder mehrfach gleich oder verschieden substituiertes Aralkyl mit 1 bis 2 Kohlenstoffatomen im Alkylteil und 6 bis 10 Kohlenstoffatomen im Arylteil steht, wobei als Arylsubstituenten jeweils infrage kommen: Halogen, geradkettiges oder verzweigtes Alkyl mit bis zu 4 Kohlenstoffatomen, sowie Halogenalkyl mit 1 oder 2 Kohlenstoffatomen und bis zu 5 gleichen oder verschiedenen Halogenatomen oder

$R^3$ und $R^4$ zusammen mit dem Stickstoffatom, an welches sie gebunden sind, für einen gegebenenfalls einfach oder mehrfach gleich oder verschieden substituierten 5- bis 7-gliedrigen gesättigten Heterocyclus mit 1 bis 3 Heteroatomen, vorzugsweise Stickstoff und Sauerstoff stehen, wobei als Substituenten infrage kommen: gerdökettiges oder verzweigtes Alkyl mit bis zu 4 Kolhenstoffatomen, geradkettiges oder verzweigtes Alkoxycarbonyl mit bis zu 5 Kohlenstoffatomen, Phenyl, Hydroxymethyl sowie die $R'-CO-O-CH_2$-Gruppe, wobei R' für geradkettiges oder verzweigtes Alkyl, Alkoxy oder Alkylamino bzw. Dialkylamino mit jeweils 1 bis 6 Kohlenstoffatomen in den einzelnen Alkylteilen, für geradkettiges oder verzweigtes Alkoxyalkyl mit jeweils 1 bis 6 Kohlenstoffatomen in beiden Alkylteilen oder für den Furylrest steht;

sowie deren pflanzenverträglichen Säureadditionssalze und Metallsalzkomplexe

2. Verbindungen der allgemeinen Formel (I) in Anspruch 1, wobei

$R^1$ für Tetrahydronaphthyl, Decahydronaphthyl oder für gegebenenfalls ein- bis dreifach, gleich oder verschieden substituiertes Naphthyl steht, wobei als Substituenten infrage kommen: Fluor, Chlor, Brom, Iod, Hydroxy, Methyl, Ethyl, Methoxy, Ethoxy, Allyloxy, Propargyloxy und Acetyloxy, weiterhin für jeweils gegebenenfalls ein- bis dreifach gleich oder verschieden durch Methyl oder Ethyl substituiertes Cycloalkyl oder Cycloalkenyl mit 5 bis 7 Kohlenstoffatomen, für gegebenenfalls ein- bis dreifach, gleich oder verschieden substituiertes Phenyl oder für einen im Phenylkern gegebenenfalls ein- bis dreifach, gleich oder verschieden substituierten Rest der Formel

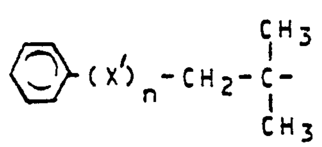 ; 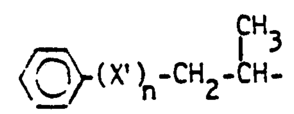 ; 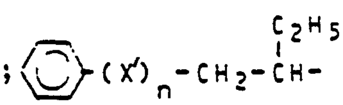 ;

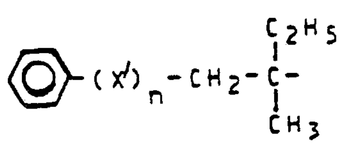 oder 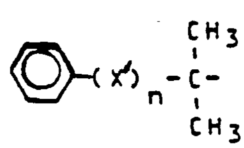 steht,

wobei als Phenylsubstituenten jeweils infrage kommen: Hydroxy, Fluor, Chlor, Brom, Iod, Cyano, Nitro, Methyl, Methoxy, Methylthio, Ethyl, Ethoxy, Ethylthio, n- und i-Propyl, Isopropyloxy, n-, i-, s- und t-Butyl, Allyloxy, Propargyloxy, Trifluormethyl, Trifluormethoxy, Trifluormethylthio, Cyclohexyl, Methoxycarbonyl, Ethoxycarbonyl, Acetyloxy, gegebenenfalls durch Fluor, Chlor oder Methyl substituiertes Phenyl oder Phenoxy, sowie der Rest R-O-N=CH-, wobei R jeweils für Methyl, Ethyl, n- und i-Propyl, n-, i-, s- und t-Butyl, Allyl und Propargyl steht; außerdem für einen im Cyclohexylteil gegebenenfalls ein- bis dreifacht gleich oder verschieden durch Methyl, Ethyl oder Isopropyl substituierten Rest der Formel

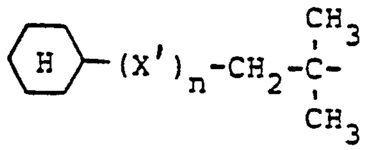 ; 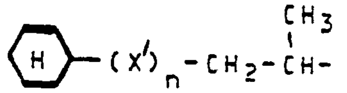 ;

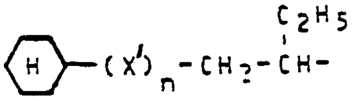 ; 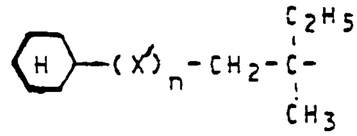 oder

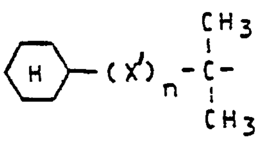 steht, wobei

in allen oben formelmäßig dargestellten Resten X' jeweils für Sauerstoff oder Schwefel und n für 0 oder 1 steht,

$R^2$ für Methyl steht,

$R^3$ für Methyl, Ethyl, n- und i-Propyl steht und

$R^4$ für Methyl, Ethyl, n- und i-Propyl n-, i-, s- und t-Butyl, Allyl, 2-Butenyl, 3-Methyl-2-butenyl sowie für gegebenenfalls ein- bis dreifach, gleich oder verschieden durch Fluor, Chlor, Methyl oder Trifluormethyl substituiertes Benzyl steht oder

$R^5$ und $R^4$ zusammen mit dem Stickstoffatom, an welches sie gebunden sind, für jeweils gegebenenfalls ein- bis dreifach, gleich oder verschieden substituiertes 1-Pyrrolidinyl, 1-Piperidinyl, 1-Piperazinyl, 4-Morpholinyl oder 1-Perhydroazepinyl stehen, wobei als Substituenten genannt seien: Methyl, Ethyl, n- und i-Propyl, Phenyl, Hydroxymethyl, Acetyloxymethyl, Methoxycarbonyloxymethyl, Dimethylaminocarbonyloxymethyl, Diethylaminocarbonyloxymethyl, Methoxyacetyloxymethyl oder Furoyloxymethyl.

3. Verfahren zur Herstellung von 5-Aminomethyl-1,3-oxathiolanen der allgemeinen Formel (I),

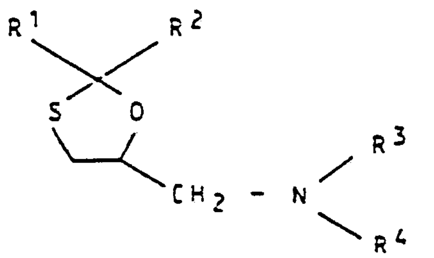 (I)

in welcher

$R^1$ für Tetrahydronaphthyl, Decahydronaphthyl oder für gegebenenfalls einfach oder mehrfach, gleich oder verschieden substituiertes Naphthyl steht, wobei als Substituenten infrage kommen: Hydroxy, Halogen, jeweils geradkettiges oder verzweigtes Alkyl, Alkoxy, Alkenyloxy, Alkinyloxy oder Alkanoyloxy mit je bis zu 4 Kohlenstoffatomen in den Alkylteilen, weiterhin für jeweils gegebenenfalls einfach oder mehrfach, gleich oder verschieden durch geradkettiges oder verzweigtes Alkyl mit bis zu 4 Kohlenstoffatomen substituiertes Cycloalkyl oder Cycloalkenyl mit je 3 bis 7 Kohlenstoffatomen steht, für gegebenenfalls einfach oder mehrfach, gleich oder verschieden substituiertes Phenyl steht, oder für im Phenylkern gegbenenfalls einfach oder mehrfach, gleich oder verschieden substituiertes Phenylalkyl, Phenoxyalkyl oder Phenylthioalkyl mit jeweils bis zu 6 Kohlenstoffatomen im geradkettigen oder verzweigten Alkylteil steht, wobei als Phenylsubstituenten jeweils infrage kommen: Hydroxy, Halogen, Cyano, Nitro, jeweils geradkettiges oder verzweigtes Alkyl, Alkoxy, Alkenyloxy, Alkinyloxy und Alkylthio mit jeweils bis zu 4 Kohlenstoffatomen, Halogenalkyl, Halogenalkoxy und Halogenalkylthio mit jeweils 1 oder 2 Kohlenstoffatomen und 1 bis 5 gleichen oder verschiedenen Halogenatomen, Cycloalkyl mit 5 bis 7 Kohlenstoffatomen, geradkettiges oder verzweigtes Alkoxycarbonyl bzw. Alkanoyloxy mit jeweils bis zu 4 Kohlenstoffatomen im Alkylteil, gegebenenfalls durch Halogen, insbesondere Fluor oder Chlor oder durch geradkettiges oder verzweigtes Alkyl mit bis zu 4 Kohlensstoffatomen substituiertes Phenyl oder Phenoxy, sowie der Rest R-O-N=CH-; wobei R jeweils für geradkettiges oder verzweigtes Alkyl, Alkenyl oder Alkinyl mit bis zu 4 Kohlenstoffatomen steht; außerdem für

jeweils im Cyclohexylteil gegebenenfalls einfach oder mehrfach, gleich oder verschieden durch geradkettiges oder verzweigtes Alkyl mit bis zu 4 Kohlenstoffatomen substituiertes Cyclohexylalkyl, Cyclohexyloxyalkyl oder Cyclohexylthioalkyl mit jeweils bis zu 6 Kohlenstoffatomen in den geradkettigen oder verzweigten Alkylteilen steht,

$R^2$ für Wasserstoff oder Methyl steht,

$R^3$ für geradkettiges oder verzweigtes Alkyl mit bis zu 4 Kohlenstoffatomen steht und

$R^4$ für geradkettiges oder verzweigtes Alkyl mit zu 4 Kohlenstoffatomen, für geradkettiges oder verzweigtes Alkenyl mit bis zu 6 Kohlenstoffatomen oder für gegebenenfalls einfach oder mehrfach gleich oder verschieden substituiertes Aralkyl mit 1 bis 2 Kohlenstoffatomen im Alkylteil und 6 bis 10 Kohlenstoffatomen im Arylteil steht, wobei als Arylsubstituenten jeweils infrage kommen: Halogen, geradkettiges oder verzweigtes Alkyl mit bis zu 4 Kohlenstoffatomen sowie Halogenalkyl mit 1 oder 2 Kohlenstoffatomen und bis zu 5 gleichen oder verschiedenen Halogenatomen oder

$R^3$ und $R^4$ zusammen mit dem Stickstoffatom, an welches sie gebunden sind, für einen gegebenenfalls einfach oder mehrfach, gleich oder verschieden substituierten 5- bis 7-gliedrigen gesättigten Heterocyclus mit 1 bis 3 Heteratomen, vorzugsweise Stickstoff und Sauerstoff stehen, wobei als Substituenten infrage kommen: geradkettiges oder verzweigtes Alkyl mit bis zu 4 Kohlenstoffatomen, geradkettiges oder verzweigtes Alkoxycarbonyl mit bis zu 5 Kohlenstoffatomen, Phenyl, Hydroxymethyl sowie die $R'-CO-OCH_2$-Gruppe, wobei R' für geradkettiges oder verzweigtes Alkyl, Alkoxy oder Alkylamino bzw. Dialkylamino mit jeweils 1 bis 6 Kohlenstoffatomen in den einzelnen Alkylteilen, für geradkettiges oder verzweigtes Alkoxyalkyl mit jeweils 1 bis 6 Kohlenstoffatomen in beiden Alkylteilen oder für den Furylrest steht,

sowie deren pflanzenverträglichen Säureadditionssalze und Metallsalzkomplexe,

dadurch gekennzeichnet, daß man in 5-Stellung substituerte 1,3-Oxathiolane der allgemeinen Formel (II),

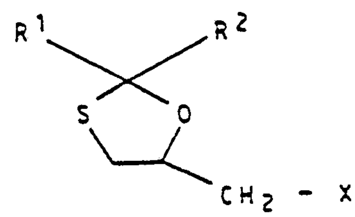

(II)

in welcher

$R^1$ und $R^2$ die oben angebene Bedeutung haben und

X für Halogen oder für gegebenfalls substituiertes Alkylsulfonyloxy bzw. Arylsulfonyloxy steht,

mit Aminen der allgemeinen Formel (III),

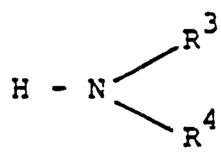

(III)

in welcher

$R^3$ und $R^4$ die oben angegebene Bedeutung haben,

gegebenenfalls in Gegenwart eines Verdünnungsmittels, gegebenenfalls in Gegenwart eines Katalysators und gegebenenfalls in Gegenwart eines Säurebindemittels umsetzt und gegebenenfalls noch anschließend eine Säure oder ein Metallsalz addiert.

4. Schädlingsbekämpfungsmittel gekennzeichnet, durch einen Gehalt an mindestens einem 5-Aminomethyl-1,3-oxathiolan der Formel (I) in Ansprüchen 1 und 3.

5. Verfahren zur Bekämpfung von Schädlingen, dadurch gekennzeichnet, daß man 5-Aminomethyl-1,3-oxathiolane der Formel (I) in Ansprüchen 1 und 3 auf Schädlinge oder ihren Lebensraum einwirken läßt, ausgenommen die durch Artikel 52(4) EPÜ verbotenen.

6. Verwendung von 5-Aminomethyl-1,3-oxathiolanen der Formel (I) in Ansprüchen 1 und 3 zur Bekämpfung von Schädlingen, soweit nach Artikel 52(4) EPÜ gewerblich anwendbar.

7. Verfahren zur Herstellung von Schädlingsbekämpfungsmitteln, dadurch gekennzeichnet, daß man 5-Amino-methyl-1,3-oxathiolane der Formel (I) in Ansprüchen 1 und 3 mit Streckmitteln und/oder oberflächenaktiven Mitteln vermischt.

8. 1,3-Oxathiolane der allgemeinen Formel (IIa)

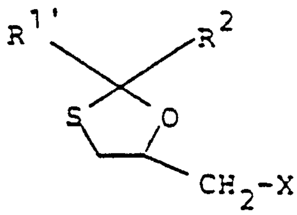

(IIa)

in welcher

$R_1$ für Tetrahydronaphthyl, Decahydronaphthyl oder für gegebenenfalls substituiertes Naphthyl, für gegebenenfalls substituiertes Cycloalkyl, für substituiertes Phenyl oder für durch - jeweils gegebenenfalls substituiertes - Phenyl, Phenoxy, Phenylthio, Cyclohexyl, Cyclohexyloxy oder Cyclohexylthio substituiertes Alkyl steht,

$R^2$ für Wasserstoff oder Methyl steht und

X für Halogen oder für gegebenenfalls substituiertes Alkylsulfonyloxy bzw. Arylsulfonyloxy steht.

9. Verfahren zur Herstellung von 1,3-Oxathiolanen der Formel (IIa)

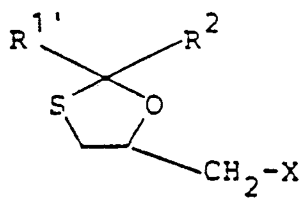

(IIa)

in welcher

$R^{1'}$ für Tetrahydronaphthyl, Decahydronaphthyl oder für gegebenenfalls substituiertes Naphthyl, für gegebenenfalls substituiertes Cycloalkyl, für substituiertes Phenyl oder für durch - jeweils gegebenenfalls substituiertes - Phenyl, Phenoxy, Phenylthio, Cyclohexyl, Cyclohexyloxy oder Cyclohexylthio substituiertes Alkyl steht,

$R^2$ für Wasserstoff oder Methyl steht und

X für Halogen oder für gegebenenfalls substituiertes Alkylsulfonyloxy bzw. Arylsulfonyloxy steht,

dadurch gekennzeichnet, daß man Aldehyde oder Ketone der Formel (IV),

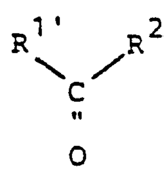

(IV)

in welcher

$R^{1'}$ und $R^2$ die oben angegebene Bedeutung haben,

mit Thiopropanol-Derivaten der Formel (V),

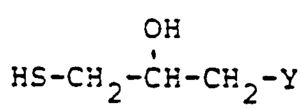

(V)

in welcher

Y für Halogen oder Hydroxy steht,

gegebenenfalls in Gegenwart eines Verdünnungsmittels und gegebenenfalls in Gegenwart eines Katalysators bei Temperaturen zwischen 0°C und 120°C umsetzt oder in den Fällen, wo Y für die Hydroxygruppe steht, die so erhaltenen 5-Hydroxy-methyl-1,3-oxathiolane der Formel (IIb),

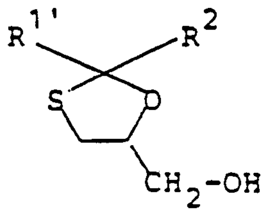

(IIb)

in welcher

$R^{1'}$ und $R^2$ die oben angegebene Bedeutüng haben,

mit gegebenenfalls substituierten Alkyl- bzw. Arylsulfonylhalogeniden der Formel (VI),

$$Z - SO_2 - Hal \qquad (VI)$$

in welcher

Hal für Halogen steht und

Z für gegebenenfalls substituiertes Alkyl bzw. Aryl, steht,

gegebenenfalls in Gegenwart eines Verdünnungsmittels, und gegebenenfalls in Gegenwart eines Säurebindemittels bei Temperaturen zwischen -20°C und +100°C umsetzt zu den Verbindungen der Formel (IIc),

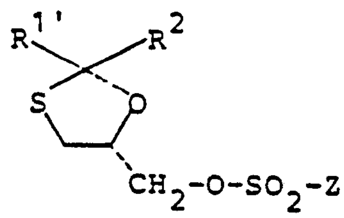

(IIc)

in welcher

$R^{1'}$, $R^2$ und Z die oben angegebene Bedeutung haben.

**Claims**

1. 5-Aminomethyl-1,3-oxathiolanes of the general formula (I)

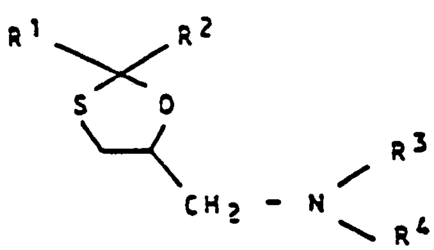

(I)

in which

$R^1$ represents tetrahydronaphthyl, decahydronaphthyl or naphthyl which is optionally monosubstituted or polysubstituted by identical or different substituents, suitable substituents being: hydroxyl, halogen or alkyl, alkoxy, alkenyloxy, alkinyloxy or alkanoyloxy, each of which is straight-chain or branched and has up to 4 carbon atoms in the alkyl parts; and furthermore represents cycloalkyl or cycloalkenyl, each of which has 3 to 7 carbon atoms and is optionally monosubstituted or polysubstituted by identical or different straight-chain or branched alkyl radicals having up to 4 carbon atoms, or represents phenyl which is optionally monosubstituted or polysubstituted by identical or different substituents, or represents phenylalkyl, phenoxyalkyl or phenylthioalkyl, each of which is optionally monosubstituted or polysubstituted in the phenyl nucleus by identical or different substituents, and has up to 6 carbon atoms in the straight-chain or branched alkyl part, suitable phenyl substituents in each case being: hydroxyl, halogen, cyano, nitro or alkyl, alkoxy, alkenyloxy, alkinyloxy and alkylthio, each of which is straight-chain or branched and has up to 4 carbon atoms,

halogenoalkyl, halogenoalkoxy and halogenoalkylthio, each of which has 1 or 2 carbon atoms and 1 to 5 identical or different halogen atoms, cycloalkyl having 5 to 7 carbon atoms, straight-chain or branched alkoxycarbonyl or alkanoyloxy, each of which has up to 4 carbon atoms in the alkyl part, phenoxy or phenyl which is optionally substituted by halogen, in particular fluorine or chlorine, or by straight-chain or branched alkyl having up to 4 carbon atoms, and the radical R-O-N=CH-, wherein R in each case represents straight-chain or branched alkyl, alkenyl or alkinyl having up to 4 carbon atoms; and furthermore represents cyclohexylalkyl, cyclohexyloxyalkyl or cyclohexylthioalkyl, each of which has up to 6 carbon atoms in the straight-chain or branched alkyl parts and is optionally monosubstituted or polysubstituted in the cyclohexyl part by identical or different, straight-chain or branched alkyl having up to 4 carbon atoms,

$R^2$ represents hydrogen or methyl,

$R^3$ represents straight-chain or branched alkyl having up to 4 carbon atoms,

and

$R^4$ represents straight-chain or branched alkyl having up to 4 carbon atoms, straight-chain or branched alkenyl having up to 6 carbon atoms or aralkyl which has 1 or 2 carbon atoms in the alkyl part and 6 to 10 carbon atoms in the aryl part and is optionally monosubstituted or polysubstituted by identical or different subsitutents, suitable aryl substituents in each case being: halogen, straight-chain or branched alkyl having up to 4 carbon aloms, and halogenoalkyl having 1 or 2 carbon atoms and up to 5 identical or different halogen atoms, or

$R^5$ and $R^4$, together with the nitrogen atom to which they are bonded, represent a 5-membered to 7-membered saturated heterocyclic structure which is optionally monosubstituted or polysubstituted by identical or different substituents and has 1 to 3 heteroatoms, preferably nitrogen or oxygen, suitable substituents being: straight-chain or branched alkyl having up to 4 carbon atoms, straight-chain or branched alkoxycarbonyl having up to 5 carbon atoms, phenyl, hydroxymethyl and the $R'$-CO-O-$CH_2$ group, wherein R' represents straight-chain or branched alkyl, alkoxy or alkylamino or dialkylamino, each having 1 to 6 carbon atoms in the individual alkyl parts, straight-chain or branched alkoxyalkyl having in each case 1 to 6 carbon atoms in the two alkyl parts, or the furyl radical, and their plant-tolerated acid addition salts and metal salt complexes.

2. Compounds of the general formula (I) in Claim 1, wherein

$R^1$ represents tetrahydronaphthyl, decahydronaphthyl or naphthyl which is optionally monosubstituted to trisubstituted by identical or different substituents, suitable substituents being: fluorine, chlorine, bromine, iodine, hydroxyl, methyl, ethyl, methoxy, ethoxy, allyloxy, propargyloxy and acetoxy; and furthermore represents cycloalkyl or cycloalkenyl, each of which is optionally monosubstituted to trisubstituted by identical or different substituents from amongst methyl or ethyl and has 5 to 7 carbon atoms, or represents phenyl which is optionally monosubstituted to trisubstituted by identical or different substituents, or represents a radical of the formula

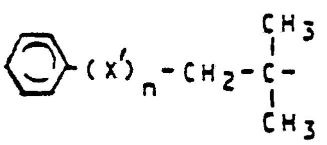 ; 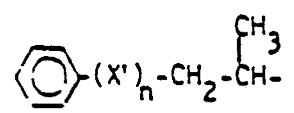 ; 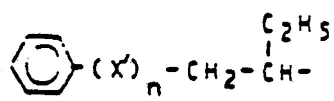 ;

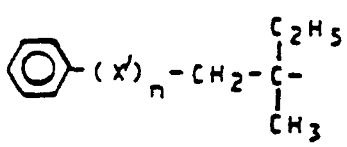 or 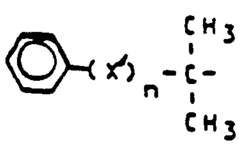

which is optionally monosubstituted to trisubstituted in the phenyl nucleus by identical or different substituents, suitable phenyl substituents in each case being: hydroxyl, fluorine, chlorine, bromine, iodine, cyano, nitro, methyl, methoxy, methylthio, ethyl, ethoxy, ethylthio, n- and i-propyl, isopropoxy, n-, i-, s- and t-butyl, allyloxy, propargyloxy, trifluoromethyl, trifluoromethoxy, trifluoromethylthio, cyclohexyl, methoxycarbonyl, ethoxycarbonyl or acetoxy, or phenoxy or phenyl which is optionally substituted by fluorine, chlorine or methyl, and the radical R-O-N=CH-, wherein R in each case represents methyl, ethyl, n-and i-propyl, n-, i-, s- and t-butyl, allyl and propargyl; and furthermore represents a radical which is optionally monosubstituted to trisubstituted in the cyclohexyl part by identical or different substituents from amongst methyl, ethyl or isopropyl, of the formula

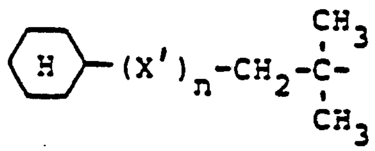 ; 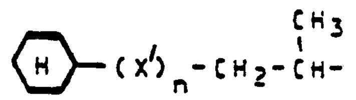 ;

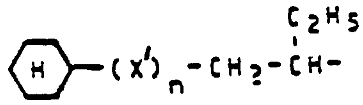 ; 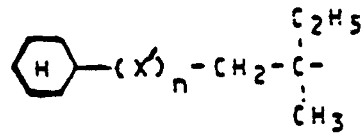 or

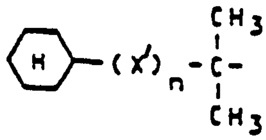

wherein, in all the radicals represented above as formulae, X' in each case represents oxygen or sulphur and n represents 0 or 1,

$R^2$ represents methyl,

$R^3$ represents methyl, ethyl, n- and i- propyl

and

$R^4$ represents methyl, ethyl, n- and i-propyl, n-, i-, s- and t-butyl, allyl, but-2-enyl or 3-methylbut-2-enyl, and represents benzyl which is optionally monosubstituted to trisubstituted by identical or different substituents from amongst fluorine, chlorine, methyl and trifluoromethyl,

or

$R^3$ and $R^4$, together with the nitrogen atom to which they are bonded, represent pyrrolidin-1-yl, piperidin-1-yl, piperazin-1-yl, morpholin-4-yl or 1-perhydroazepinyl, each of which is optionally monosubstituted to trisubstituted by identical or different substituents, the following being mentioned as substituents: methyl, ethyl, n- and i-propyl, phenyl, hydroxymethyl, acetoxymethyl, methoxycarbonyloxymethyl, dimethylaminocarbonyloxymethyl, diethylaminocarbonyloxymethyl, methoxyacetoxymethyl or furoyloxymethyl.

3. Process for the preparation of 5-aminomethyl-1,3-oxathiolanes of the general formula (I)

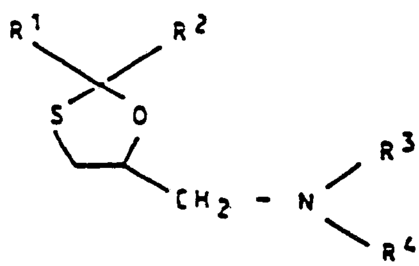 (I)

in which

$R^1$ represents tetrahydronaphthyl, decahydronaphthyl or naphthyl which is optionally monosubstituted or polysubstituted by identical or different substituents, suitable substituents being: hydroxyl, halogen or alkyl, alkoxy, alkenyloxy, alkinyloxy or alkanoyloxy, each of which is straight-chain or branched and has up to 4 carbon atoms in the alkyl parts; and furthermore represents cycloalkyl or cycloalkenyl, each of which has 3 to 7 carbon atoms and is optionally monosubstituted or polysubstituted by idenlical or different straight-chain or branched alkyl radicals having up to 4 carbon atoms, or represents phenyl which is optionally monosubstituted or polysubstituted by identical or different substituents, or represents phenylalkyl, phenoxyalkyl or phenylthioalkyl, each of which is optionally monosubstituted or polysubstituted in the phenyl nucleus by identical or different substituents, and has up to 6 carbon atoms in the straight-chain or branched alkyl part, suitable phenyl substituents in each case being: hydroxyl, halogen, cyano, nitro or alkyl, alkoxy, alkenyloxy, alkinyloxy and alkylthio, each of which is straight-chain or branched and has up to 4 carbon atoms, halogenoalkyl, halogenoalkoxy and halogenoalkylthio, each of which has 1 or 2 carbon atoms and 1 to 5 identical or different halogen atoms, cycloalkyl having 5 to 7 carbon atoms, straight-chain or branched alkoxycarbonyl or alkanoyloxy, each of which has up to 4 carbon atoms in the alkyl part, phenoxy or phenyl which is optionally substituted by halogen, in particular fluorine or chlorine, or by straight-chain or branched alkyl having up to 4 carbon atoms, and the radical R-O-N=CH-, wherein R in each case represents straight-chain or branched alkyl, alkenyl or alkinyl having up to 4 carbon atoms; and furthermore represents cyclohexylalkyl, cyclohexyloxyalkyl or cyclohexylthioalkyl, each of which has up to 6 carbon atoms in the

straight-chain or branched alkyl parts and is optionally monosubstituted or polysubstituted in the cyclohexyl part by identical or different, straight-chain or branched alkyl having up to 4 carbon atoms,

$R^2$ represents hydrogen or methyl,

$R^3$ represents straight-chain or branched alkyl having up to 4 carbon atoms,

and $R^4$ represents straight-chain or branched alkyl having up to 4 carbon atoms, straight-chain or branched alkenyl having up to 6 carbon atoms or aralkyl which has 1 or 2 carbon atoms in the alkyl part and 6 to 10 carbon atoms in the aryl part and is optionally monosubstituted or polysubstituted by identical or different substituents, suitable aryl substituents in each case being: halogen, straight-chain or branched alkyl having up to 4 carbon atoms, and halogenoalkyl having 1 or 2 carbon atoms and up to 5 identical or different halogen atoms, or

$R^3$ nd $R^4$, together with the nitrogen atom to which they are bonded, represent a 5-membered to 7-membered saturated heterocyclic structure which is optionally monosubstituted or polysubstituted by identical or different substituents and has 1 to 3 heteroatoms, preferably nitrogen or oxygen, suitable substituents being: straight-chain or branched alkyl having up to 4 carbon atoms, straight-chain or branched alkoxycarbonyl having up to 5 carbon atoms, phenyl, hydroxymethyl and the $R'-CO-O-CH_2$-group, wherein R' represents straight-chain or branched alkyl, alkoxy or alkylamino or dialkylamino, each having 1 to 6 carbon atoms in the individual alkyl parts, straight-chain or branched alkoxyalkyl having in each case 1 to 6 carbon atoms in the two alkyl parts, or the furyl radical, and their plant-tolerated acid addition salts and metal salt complexes,

characterized in that 1,3-oxathiolanes which are substituted in the 5-position, of the general formula (II)

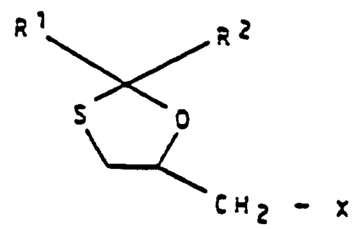

(II)

in which

$R^1$ and $R^2$ have the meaning given above and

X represents halogen or optionally substituted alkylsulphonyloxy or arylsulphonyloxy, are reacted with amines of the general formula (III)

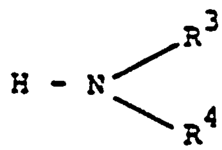

(III)

in which

$R^3$ and $R^4$ have the meaning given above, if appropriate in the presence of a diluent, if appropriate in the presence of a catalyst and, if appropriate, in the presence of an acid-binding agent, and, if required, the product is then subjected to an addition reaction with an acid or a metal salt.

4. Pest-combating agents, characterized in that they contain at least one 5-aminomethyl-1,3-oxathiolane of the formula (I) in claims 1 and 3.

5. Method of combating pests, characterized in that 5-aminomethyl-1,3-oxathiolanes of the formula (I) in Claims 1 and 3 are allowed to act on pests or their habitat, with the exception of those prohibited under Article 52(4) EPC.

6. Use of 5-aminomethyl-1,3-oxathiolanes of the formula (I) in Claims 1 and 3 for combating pests, as far as is commercially practicable according to Article 52(4) EPC.

7. Process for the preparation of pest-combating agents, characterized in that 5-aminomethyl-1,3-oxathiolanes of the formula (I) in Claims 1 and 3 are mixed with extenders and/or surface-active agents.

8. 1,3-Oxathiolanes of the general formula (IIa)

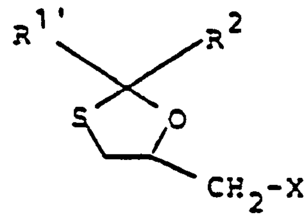

(IIa)

in which

$R^{1'}$ represents tetrahydronaphthyl, decahydronaphthyl or optionally substituted naphthyl, optionally substituted cycloalkyl, substituted phenyl, or alkyl which is substituted by phenyl, phenoxy, phenylthio, cyclohexyl, cyclohexyloxy or cyclohexylthio, each of which is optionally substituted,

$R^2$ represents hydrogen or methyl, and

X represents halogen or optionally substituted alkylsulphonyloxy or arylsulphonyloxy.

9. Process for the preparation of 1,3-oxathiolanes of the formula (IIa)

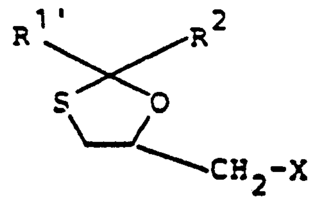

(IIa)

in which

$R^{1'}$ represents tetrahydronaphthyl, decahydronaphthyl or optionally substituted naphthyl, optionally substituted cycloalkyl, substituted phenyl, or alkyl which is substituted by phenyl, phenoxy, phenylthio, cyclohexyl, cyclohexyloxy or cyclohexylthio, each of which is optionally substituted,

$R^2$ represents hydrogen or methyl, and

X represents halogen or optionally substituted alkylsulphonyloxy or arylsulphonyloxy,

characterized in that aldehydes or ketones of the formula (IV)

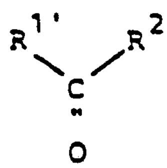

(IV)

in which

$R^{1'}$ and $R^2$ have the meaning given above, are reacted with thiopropanol derivatives of the formula (V)

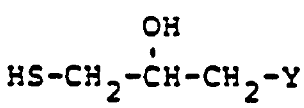

(V)

in which

Y represents halogen or hydroxyl,

if appropriate in the presence of a diluent and, if appropriate, in the presence of a catalyst, at temperatures between 0°C and 120°C, or, in the cases where Y represents the hydroxyl group, the resulting 5-hydroxymethyl-1,3-oxathiolanes of the formula (IIb)

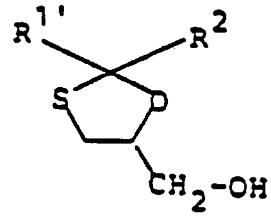

(IIb)

in which

$R^{1'}$ and $R^2$ have the meaning given above, are reacted with optionally substituted alkyl- or arylsulphonyl halides of the formula (VI)

$$Z - SO_2 - Hal \qquad (VI)$$

in which

Hal represents halogen and

Z represents optionally substituted alkyl or aryl,

if appropriate in the presence of a diluent, and, if appropriate, in the presence of an acid-binding agent, at temperatures between -20°C and +100°C, to give the compounds of the formula (IIc)

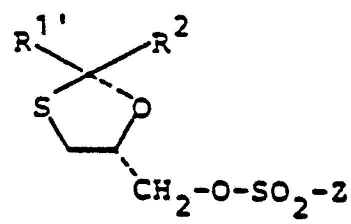

(IIc)

in which

$R^{1'}$, $R^2$ and Z have the meaning given above.

## Revendications

1. 5-aminométhyl-1,3-oxazthiolanes de formule générale (I)

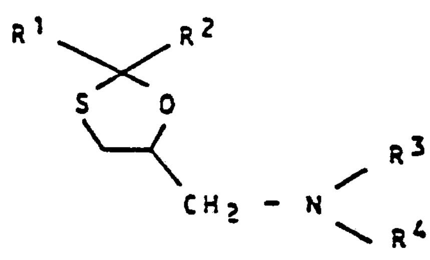

(I)

dans laguelle

$R^1$ est un groupe tétrahydronaphtyle, décahydronaphtyle ou un groupe naphtyle portant éventuellement un ou plusieurs substituants identiques ou différents, et on considère alors comme substituants les substituants suivants: hydroxy, halogéno, alkyle, alkoxy, alcényloxy, alcynyloxy ou alcanoyloxy, chacun à chaîne droite ou à chaîne ramifiée avec jusgu'à 4 atomes de carbone dans les parties alkyle, en outre un groupe cycloalkyle ou un groupe cycloalcényle ayant chacun 3 à 7 atomes de carbone, portant chacun éventuellement un ou plusieurs substituants alkyle à chaîne droite ou ramifiée, identiques ou différents, ayant jusgu'à 4 atomes de carbone, un groupe phényle portant éventuellement un ou plusieurs substituants identiques ou différents, ou un groupe phénylalkyle, phénoxyalkyle ou phénylthioalkyle portant éventuellement un ou plusieurs substituants identiques ou différents dans le noyau phényle, avec chacun jusqu'à 6 atomes de carbone dans la partie alkyle à chaîne droite ou à chaîne ramifiée, et on considère alors dans chaque cas comme substituants du groupe phényle : un substituant hydroxy, halogéno, cyano, nitro, un substituant alkyle, alkoxy, alcényloxy, alcynyloxy ou alkylthio à chaîne droite ou ramifiée avec dans chaque cas jusqu'à 4 atomes de carbone, halogénalkyle,

halogénalkoxy et halogénalkylthio ayant chacun un ou deux atomes de carbone et 1 à 5 atomes d'halogènes identiques ou différents, un groupe cycloalkyle de 5 à 7 atomes de carbone, un groupe alkoxycarbonyle ou alcanoyloxy à chaîne droite ou à chaîne ramifiée ayant chacun jusqu'à 4 atomes de carbone dans la partie alkyle, un groupe phényle ou phénoxy éventuellement substitué par un halogène, notamment le fluor ou le chlore, ou par un radical alkyle à chaîne droite ou à chaîne ramifiée ayant jusqu'à 4 atomes de carbone, ainsi que le reste R-O-N=CH-, dans lequel R représente dans chaque cas un groupe alkyle, alcényle ou alcynyle à chaîne droite ou à chaîne ramifiée ayant jusqu'à 4 atomes de carbone, en outre un groupe cyclohexylalkyle, cyclohexyloxyalkyle ou cyclohexylthioalkyle ayant chacun jusqu'à 6 atomes de carbone dans les parties alkyle à chaîne droite ou à chaîne ramifiée, éventuellement substitués chacun dans la partie cyclohexyle par un ou plusieurs substituants alkyle à chaîne droite ou à chaîne ramifiée, identiques ou différents, ayant jusqu'à 4 atomes de carbone,

$R^2$ est l'hydrogène ou le groupe méthyle,

$R^3$ est un groupe alkyle à chaîne droite ou à chaîne ramifiée ayant jusqu'à 4 atomes de carbone et

$R^4$ est un groupe alkyle à chaîne droite ou à chaîne ramifiée ayant jusqu'à 4 atomes de carbone, un groupe alcényle à chaîne droite ou à chaîne ramifiée ayant jusqu'à 6 atomes de carbone ou un groupe aralkyle portant éventuellement un ou plusieurs substituants identiques ou différents, avec 1 ou 2 atomes de carbone dans la partie alkyle et 6 à 10 atomes de carbone dans la partie aryle, et on considère alors dans chaque cas comme substituants de la partie aryle un: halogène, un groupe alkyle à chaîne droite ou à chaîne ramifiée ayant jusqu'à 4 atomes de carbone ainsi qu'un groupe halogénalkyle ayant 1 ou 2 atomes de carbone et jusqu'à 5 atomes d'halogènes identiques ou différents, ou bien

$R^3$ et $R^4$ forment, conjointement avec l'atome d'azote auquel ils sont liés, un hétérocycle saturé pentagonal à heptagonal portant éventuellement un ou plusieurs substituants identiques ou différents, avec 1 à 3 hétéroatomes, de préférence d'azote et d'oxygène, et on considère alors comme substituants un groupe alkyle à chaîne droite ou à chaîne ramifiée ayant jusqu'à 4 atomes de carbone, un groupe alkoxycarbonyle à chaîne droite ou à chaîne ramifiée ayant jusqu'à 5 atomes de carbone, un groupe phényle, hydroxyméthyle ainsi que le groupe $R'-CO-O-CH_2-$ dans lequel R' est un groupe alkyle, alkoxy ou alkylamino ou dialkylamino à chaîne droite ou ramifiée avec dans chaque cas 1 à 6 atomes de carbone dans les parties alkyle individuelles, un groupe alkoxyalkyle à chaîne droite ou ramifiée ayant 1 à 6 atomes de carbone dans les deux parties alkyle, ou le reste furyle,

ainsi que leurs sels d'addition d'acides et leurs complexes de sels métalliques compatibles avec les plantes.

2. Composés de formule générale (I) suivant la revendication 1, dans lesquels

$R^1$ est un groupe tétrahydronaphtyle, décahydronaphtyle ou un groupe naphtyle portant éventuellement 1 à 3 substituants identiques ou différents, et on considère alors comme substituants: le fluor, le chlore, le brome, l'iode, un groupe hydroxy, méthyle, éthyle, méthoxy, éthoxy, allyloxy, propargyloxy et acétyloxy, en outre, un groupe cycloalkyle ou cycloalcényle de 5 à 7 atomes de carbone portant éventuellement 1 à 3 substituants méthyle ou éthyle identiques ou différents, un groupe phényle portant éventuellement 1 à 3 substituants identiques ou différents ou un reste, portant éventuellement 1 à 3 substituants identiques ou différents dans le noyau phényle, de formule:

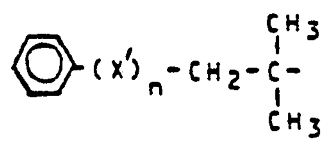 ; 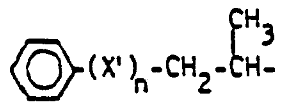 ; 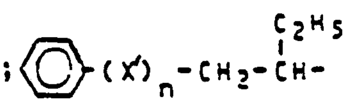 ;

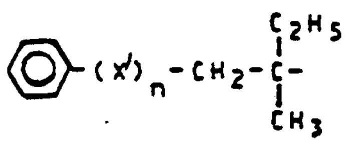 ou 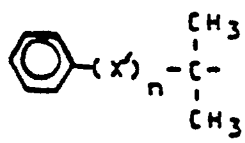 ,

et on considère alors dans chaque cas comme substituants du groupe phényle: un substituant hydroxy, fluor, chlore, brome, iode, cyano, nitro, méthyle, méthoxy, méthylthio, éthyle, éthoxy, éthylthio, n-propyle, isopropyle, isopropyloxy, n-butyle, isobutyle, sec.-butyle, tertio-butyle, allyloxy, propargyloxy, trifluorométhyle, trifluorométhoxy, trifluorométhylthio, cyclohexyle, méthoxycarbonyle, éthoxycarbonyle, acétyloxy, phényle ou phénoxy éventuellement substitué par du fluor, du chlore ou un radical méthyle, ainsi que le reste R-O-N=CH- dans lequel R représente dans chague cas un radical méthyle, éthyle, n-propyle, isopropyle, n-butyle, isobutyle, sec.-butyle et tertiobutyle, allyle et propargyle, en outre, un reste, portant éventuellement 1 à 3 substituants méthyle, éthyle ou isopropyle identiques ou différents dans la partie cyclohexyle, de formule:

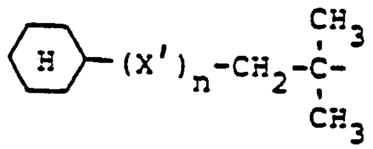 ; 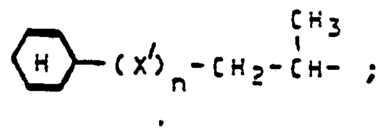 ;

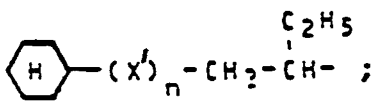 ; 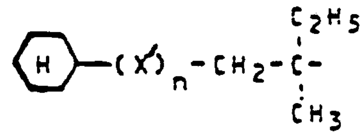 ou

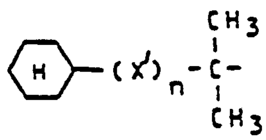

auquel cas dans tous les restes représentés ci-dessus par des formules, X' désigne dans chaque cas de l'oxygène ou du soufre et n a la valeur zéro ou un,

$R^2$ est un groupe méthyle,

$R^3$ est un groupe méthyle, éthyle, n-propyle et isopropyle et

$R^4$ est un groupe méthyle, éthyle, n-propyle, isopropyle, n-butyle, isobutyle, sec.-butyle, tertio-butyle, allyle, 2-buténylе, 3-méthyl-2-butényle, ainsi qu'un groupe benzyle portant éventuellement 1 à 3 substituants fluoro, chloro, méthyle ou trifluorométhyle identiques ou différents, ou bien

$R^3$ et $R^4$ forment, conjointement avec l'atome d'azote auquel ils sont liés, un reste 1-pyrrolidinyle, 1-pipéridinyle, 1-pipérazinyle, 4-morpholinyle ou 1-perhydroazépinyle portant chacun éventuellement 1 à 3 substituants identiques ou différents, et on mentionne alors comme substituants les substituants suivants: méthyle, éthyle, n-propyle, isopropyle, phényle, hydroxyméthyle, acétyloxyméthyle, méthoxycarbonyloxyméthyle, diméthylaminocarbonyloxyméthyle, diéthylaminocarbonyloxyméthyle, méthoxyacétyloxyméthyle ou furoyloxyméthyle.

3. Procédé de production de 5-aminométhyl-1,3-oxathiolanes de formule générale (I):

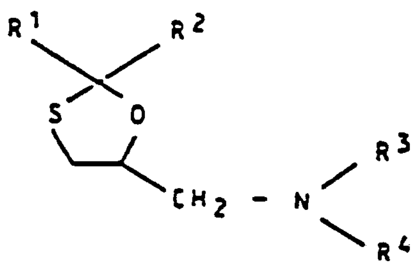 (I)

dans laquelle

$R^1$ est un groupe tétrahydronaphtyle, décahydronaphtyle ou un groupe naphtyle portant éventuellement un ou plusieurs substituants identiques ou différents, et on considère alors comme substituants les substituants suivants: hydroxy, halogéno, alkyle, alkoxy, alcényloxy, alcynyloxy ou alcanoyloxy, chacun à chaîne droite ou à chaine ramifiée avec jusqu'à 4 atomes de carbone dans les parties alkyle, en outre un groupe cycloalkyle ou un groupe cycloalcényle ayant chacun 3 à 7 atomes de carbone, portant chacun éventuellement un ou plusieurs substituants alkyle à chaîne droite ou ramifiée, identiques ou différents, ayant jusqu'à 4 atomes de carbone, un groupe phényle portant éventuellement un ou plusieurs substituants identiques ou différents, ou un groupe phénylalkyle, phénoxyalkyle ou phénylthioalkyle portant éventuellement un ou plusieurs substituants identiques ou différents dans le noyau phényle, avec chacun jusqu'à 6 atomes de carbone dans la partie alkyle à chaîne droite ou à chaîne ramifiée, et on considère alors dans chaque cas comme substituants du groupe phényle: un substituant hydroxy, halogéno, cyano, nitro, un substituant alkyle, alkoxy, alcényloxy, alcynyloxy ou alkylthio à chaîne droite ou ramifiée avec dans chaque cas jusqu'à 4 atomes de carbone, halogénalkyle, halogénalkoxy et halogénylalkylthio ayant chacun un ou deux atomes de carbone et 1 à 5 atomes d'halogènes identiques ou différents, un groupe cycloalkyle de 5 à 7 atomes de carbone, un groupe alkoxycarbonyle ou alcanoyloxy à chaîne droite ou à chaîne ramifiée ayant chacun jusqu'à 4 atomes de carbone dans la partie alkyle, un groupe phényle ou phénoxy éventuellement substitué par un halogène, notamment le fluor ou le

chlore, ou par un radical alkyle à chaîne droite ou à chaîne ramifiée ayant jusqu'à 4 atomes de carbone, ainsi que le reste R-O-N=CH-, dans lequel R représente dans chaque cas un groupe alkyle, alcényle ou alcynyle à chaîne droite ou à chaîne ramifiée ayant jusqu'à 4 atomes de carbone, en outre un groupe cyclohexylalkyle, cyclohexyloxyalkyle ou cyclohexylthioalkyle ayant chacun jusqu'a 6 atomes de carbone dans les parties alkyle à chaîne droite ou à chaîne ramifiée, éventuellement substitués chacun dans la partie cyclohexyle ou par un ou plusieurs substituants alkyle à chaîne droite ou à chaîne ramifiée, identiques ou différents, ayant jusqu'à 4 atomes de carbone,

$R^2$ est l'hydrogène ou le groupe méthyle,

$R^3$ est un groupe alkyle à chaîne droite ou à chaîne ramifiée ayant jusqu'à 4 atomes de carbone et

$R^4$ est un groupe alkyle à chaîne droite ou à chaîne ramifiée ayant jusqu'à 4 atomes de carbone, un groupe alcényle à chaîne droite ou à chaîne ramifiée ayant jusqu'à 6 atomes de carbone ou un groupe aralkyle portant éventuellement un ou plusieurs substituants identiques ou différents, avec 1 ou 2 atomes de carbone dans la partie alkyle et 6 à 10 atomes de carbone dans la partie aryle, et on considère alors dans chaque cas comme substituants de la partie aryle : un halogène, un groupe alkyle à chaîne droite ou à chaîne ramifiée ayant jusqu'à 4 atomes de carbone ainsi qu'un groupe halogénalkyle ayant 1 ou 2 atomes de carbone et jusqu'à 5 atomes d'halogènes identiques ou différents, ou bien

$R^3$ et $R^4$ forment, conjointement avec l'atome d'azote auquel ils sont liés, un hétérocycle saturé pentagonal à heptagonal portant éventuellement un ou plusieurs substituants identiques ou différents, avec 1 à 3 hétéroatomes, de préférence d'azote et d'oxygène, et on considère alors comme substituants un groupe alkyle à chaîne droite ou à chaîne ramifiée ayant jusqu'à 4 atomes de carbone, un groupe alkoxycarbonyle à chaîne droite ou à chaîne ramifiée ayant jusqu'à 5 atomes de carbone, un groupe phényle, hydroxyméthyle ainsi que le groupe $R'-CO-O-CH_2-$ dans lequel R' est un groupe alkyle, alkoxy ou alkylamino ou dialkylamino à chaîne droite ou ramifiée avec dans chaque cas 1 à 6 atomes de carbone dans les parties alkyle individuelles, un groupe alkoxyalkyle à chaîne droite ou ramifiée ayant 1 à 6 atomes de carbone dans les deux parties alkyle, ou le reste furyle,

ainsi que de leurs sels d'addition d'acides et de leurs complexes de sels métalliques compatibles avec les plantes, caractérisé en ce qu'on fait réagir des 1,3-oxathiolanes substitués en position 5 de formule générale (II)

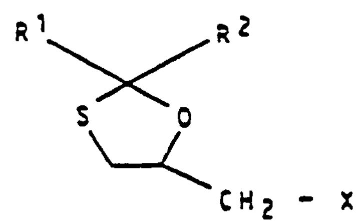

(II)

dans laquelle

$R^1$ et $R^2$ ont la définition indiquée ci-dessus et

X représente un halogène ou un groupe alkylsulfonyloxy ou arylsulfonyloxy éventuellement substitué,

avec des amines de formule générale (III)

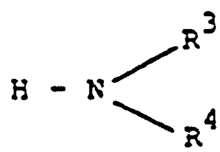

(III)

dans laquelle

$R^3$ et $R^4$ ont la définition indiquée ci-dessus

le cas échéant en présence d'un diluant, éventuellement en présence d'un catalyseur et le cas échéant en présence d'un accepteur d'acide, puis on additionne encore le cas échéant un acide ou un sel métallique.

4. Compositions pesticides, caractérisées par une teneur en au moins un 5-aminométhyl-1,3-oxathiolane de formule (I) suivant les revendications 1 et 3.

5. Procédé pour combattre les parasites, caractérisé en ce qu'on fait agir des 5-aminométhyl-1,3-oxathiolanes de formule (I) suivant les revendications 1 et 3 sur les parasites ou sur leur habitat, excepté ceux qui sont interdits par l'article 52(4) de la convention sur le brevet européen.

6. Utilisation de 5-aminométhyl-1,3-oxathiolanes de formules (I) suivant les revendications 1 et 3 pour combattre des parasites, dans la mesure où leur usage professionnel est permis d'après l'article 52(4) de la convention sur le brevet européen.

7. Procédé de production de compositions pesticides, caractérisé en ce qu'on mélange des 5-aminométhyl-1,3-oxathiolanes de formule (I) suivant les revendications 1 et 3 avec des diluants et/ou des agents tensioactifs.

8. 1,3-oxathiolanes de formule générale (IIa) dans laquelle

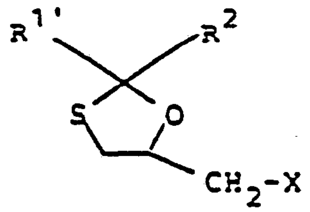

(IIa)

$R^{1'}$ est un groupe tétrahydronaphtyle, décahydronaphtyle ou un groupe naphtyle éventuellement substitué, cycloalkyle éventuellement substitué, phényle substitué ou un groupe alkyle substitué par un radical phényle, phénoxy, phénylthio, cyclohexyle, cyclohexyloxy ou cyclohexylthio, chacun substitué le cas échéant,

$R^2$ est l'hydrogène ou le groupe méthyle et

X est un halogène ou un reste alkylsulfonyloxy ou arylsulfonyloxy éventuellement substitué.

9. Procédé de production de 1,3-oxathiolanes de formule (IIa)

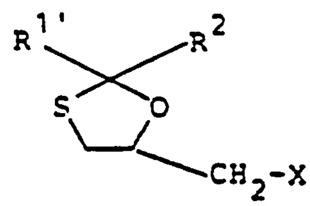

(IIa)

dans laquelle

$R^{1'}$est un groupe tétrahydronaphtyle, décahydronaphtyle ou un groupe naphtyle éventuellement substitué, un groupe cycloalkyle éventuellement substitué, un groupe phényle substitué ou un groupe alkyle substitué par un radical phényle, phénoxy, phénylthio, cyclohexyle, cyclohexyloxy ou cyclohexylthio, chacun éventuellement substitué,

$R^2$ est l'hydrogène ou un groupe méthyle et

X est un halogène ou un groupe alkylsulfonyloxy ou arylsulfonyloxy éventuellement substitué,

caractérisé en ce qu'on fait réagir des aldéhydes ou des cétones de formule (IV)

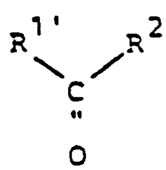

(IV)

dans laquelle

$R^{1'}$et $R^2$ ont la définition indiquée ci-dessus, avec des dérivés de thiopropanols de formule (V)

$$HS-CH_2-\underset{}{\overset{OH}{\overset{|}{C}H}}-CH_2-Y \qquad (V)$$

dans laquelle

Y est un halogène ou un groupe hydroxy,

éventuellement en présence d'un diluant et, le cas échéant, en présence d'un catalyseur à des températures comprises entre 0 et 120°C ou bien, au cas où Y représente le groupe hydroxy, les 5-hydroxyméthyl-1,3-oxathiolanes de formule (IIb) ainsi obtenus

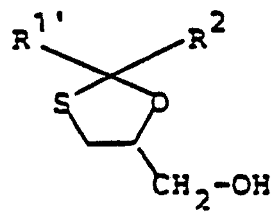

(IIb)

formule dans laquelle

$R^{1'}$ et $R^2$ ont la définition indiquée ci-dessus, sont transformés par réaction avec des halogénures d'alkyl-ou d'arylsulfonyle éventuellement substitués de formule (VI)

$$Z - SO_2 - Hal \qquad (VI)$$

dans laquelle
Hal est un halogène et
Z est un groupe alkyle ou aryle éventuellement substitué
éventuellement en présence d'un diluant et, le cas échéant, en présence d'un accepteur d'acide, à des températures comprises entre -20°C et +100°C, en composés de formule (IIc)

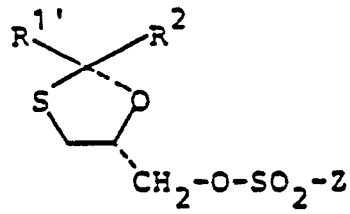

(IIc)

dans laquelle
$R^{1'}$, $R^2$ et Z ont la définition indiquée ci-dessus.